(19) Europäisches Patentamt
European Patent Office
Office européen des brevets

(11) EP 1 742 056 A2

(12) **EUROPÄISCHE PATENTANMELDUNG**

(43) Veröffentlichungstag:
**10.01.2007 Patentblatt 2007/02**

(21) Anmeldenummer: **06007308.7**

(22) Anmeldetag: **24.07.1995**

(51) Int Cl.:
***G01N 33/533*** *(2006.01)* ***G01N 33/58*** *(2006.01)*
***C07F 15/00*** *(2006.01)* ***C07K 14/16*** *(2006.01)*
***G01N 33/543*** *(2006.01)* ***G01N 33/68*** *(2006.01)*
***G01N 33/577*** *(2006.01)*

(84) Benannte Vertragsstaaten:
**AT BE CH DE DK ES FR GB IE IT LI NL SE**

(30) Priorität: **25.07.1994 DE 4426276**
**31.08.1994 DE 4430972**

(62) Dokumentnummer(n) der früheren Anmeldung(en) nach Art. 76 EPÜ:
**95928450.6 / 0 772 774**

(71) Anmelder: **Roche Diagnostics GmbH**
**68305 Mannheim (DE)**

(72) Erfinder:
- **Wienhues-Thelen, Ursula-Henrike, Dr.**
  **82152 Krailling (DE)**
- **Kruse-Mueller, Cornelia, Dr.**
  **Danbury, CT 06810 (US)**
- **Hoess, Eva, Dr.**
  **82061 Neuried (DE)**
- **Ofenloch-Haehnle, Beatus, Dr.**
  **82398 Polling (DE)**
- **Seidel, Christoph, Dr.**
  **82362 Weilheim (DE)**
- **Faatz, Elke, Dr.**
  **82386 Huglfing (DE)**
- **Wiedmann, Michael, Dr.**
  **82377 Penzberg (DE)**

(74) Vertreter: **Weiss, Wolfgang et al**
**Weickmann & Weickmann**
**Patentanwälte**
**Postfach 86 08 20**
**81635 München (DE)**

Bemerkungen:
Diese Anmeldung ist am 06 - 04 - 2006 als Teilanmeldung zu der unter INID-Kode 62 erwähnten Anmeldung eingereicht worden.

(54) **Bestimmung von spezifischem Immunglobulin unter Verwendung multipler Antigene**

(57) Die Erfindung betrifft ein Verfahren zur immunologischen Bestimmung eines spezifischen Antikörpers in einer Probeflüssigkeit, wobei man die Probeflüssigkeit in Gegenwart einer Festphase mit zwei gegen den zu bestimmenden Antikörper gerichteten Antigenen inkubiert, wobei das erste Antigen mindestens eine Markierungsgruppe trägt und das zweite Antigen (a) an die Festphase gebunden ist oder (b) in einer an die Festphase bindefähigen Form vorliegt, und den zu bestimmenden Antikörper durch Bestimmung der Markierungsgruppe in der Festphase oder/und in der flüssigen Phase nachweist, welches dadurch gekennzeichnet ist, daß mindestens eines der beiden Antigene mehrere Epitopbereiche umfaßt, die mit dem zu bestimmenden Antikörper reagieren.



Printed by Jouve, 75001 PARIS (FR)

**Beschreibung**

Die vorliegende Erfindung betrifft ein Verfahren zur Bestimmung von spezifischem Immunglobulin unter Verwendung von Antigenen, die mehrere Epitopbereiche umfassen.

Der Nachweis von Immunglobulinen in Körperflüssigkeiten, insbesondere in Humanseren, wird zur Diagnostik von Infektionen mit Mikroorganismen, insbesondere Viren, wie etwa HIV, Hepatitis-Viren, etc. verwendet. Das Vorhandensein von spezifischen Immunglobulinen in der untersuchten Probe wird üblicherweise durch Reaktion mit einem oder mehreren Antigenen, die mit den spezifischen Immunglobulinen reagieren, nachgewiesen. Verfahren zur Bestimmung von spezifischen Immunglobulinen in der Probeflüssigkeit müssen sensitiv, zuverlässig, einfach und schnell sein.

In den letzten Jahren wurden zunehmend Nachweissysteme auf Basis nicht-radioaktiver Markierungsgruppen entwickelt, bei denen das Vorhandensein eines Analyten, z.B. eines spezifischen Antikörpers, in der untersuchten Probe mit Hilfe optischer (z.B. Lumineszenz oder Fluoreszenz), NMR-aktiver oder Metallpräzipitierender Detektionssysteme bestimmt werden konnte.

EP-A-0 307 149 offenbart einen Immuntest für einen Antikörper, bei dem zwei rekombinante Polypeptide als Antigene verwendet werden, von denen eines an einer festen Phase immobilisiert ist, und das andere eine Markierungsgruppe trägt, wobei beide rekombinanten Antigene in unterschiedlichen Organismen exprimiert werden, um die Spezifität des Nachweises zu erhöhen.

EP-A-0 366 673 offenbart ein Verfahren zum Nachweis von Antikörpern in einer Probe, bei dem ein Antikörper durch Reaktion mit einem gereinigten, markierten Antigen und dem gleichen gereinigten Antigen in einer Festphasen-gebundenen Form nachgewiesen wird. Als Antigen wird beispielsweise humanes IgG offenbart.

EP-A-0 386 713 beschreibt ein Verfahren zum Nachweis von Antikörpern gegen HIV unter Verwendung von zwei festen Trägern, wobei an beide festen Träger verschiedene HIV-Antigene immobilisiert werden, die jeweils mit einem Aliquot einer Probe und einem markierten HIV-Antigen in Kontakt gebracht werden, wobei das Vorhandensein von Antikörpern durch eine positive Reaktion in mindestens einem der Tests nachgewiesen wird. Als HIV-Antigene werden rekombinant hergestellte Polypeptide offenbart.

EP-A-0 507 586 beschreibt ein Verfahren zur Durchführung eines immunologischen Tests für ein spezifisches Immunglobulin, bei dem eine Probe mit zwei zur Bindung des Immunglobulins fähigen Antigenen in Kontakt gebracht wird, wobei das erste Antigen eine zur Bindung an einen festen Träger geeignete Gruppe trägt, und das zweite Antigen eine Markierungsgruppe trägt. Die Markierungsgruppe kann eine direkte Markierungsgruppe sein, z.B. ein Enzym, ein Chromogen, ein Metallteilchen, oder auch eine indirekte Markierungsgruppe, d.h. die am Antigen angebrachte Markierungsgruppe kann mit einem Rezeptor für die Markierungsgruppe, der wiederum eine signalerzeugende Gruppe trägt, reagieren. Als Beispiel für eine solche indirekte Markierungsgruppe wird ein Fluoresceinderivat genannt, dessen Rezeptor ein Antikörper ist, der wiederum mit einem Enzym gekoppelt ist. Als Antigene werden Polypeptide, wie etwa das Hepatitis B-Oberflächenantigen offenbart. In dieses Antigen werden durch Derivatisierung SH-Gruppen eingeführt, mit denen das Fluorescein gekoppelt wird.

EP-A-0 507 587 offenbart ein spezifisch zum Nachweis von IgM Antikörpern geeignetes Verfahren, bei dem die Probe mit einem markierten Antigen, das gegen den nachzuweisenden Antikörper gerichtet ist, und einem zweiten Antikörper, der ebenfalls gegen den nachzuweisenden Antikörper gerichtet und an eine Festphase bindefähig ist, inkubiert wird.

Die aus dem Stand der Technik bekannten immunologischen Nachweisverfahren nach dem Brückentestkonzept, bei denen ein markiertes Antigen und ein an eine Festphase bindefähiges Antigen verwendet wird, weisen jedoch noch erhebliche Schwächen auf. Insbesondere findet man eine geringe Sensitivität, wenn zwischen dem zu bestimmenden Antikörper und dem Antigen eine relativ geringe Affinität vorliegt. Dies ist insbesondere bei einer erst seit kurzem erfolgten Serokonversion und/oder beim Auftreten neuer Subtypen des infektiösen Mikroorganismus der Fall. Ein weiterer Nachteil der bisher bekannten Brückentestkonzepte besteht im Risiko einer falsch negativen Bewertung hochtitriger Proben aufgrund des Hook-Effekts.

Das der vorliegenden Erfindung zugrundeliegende Problem war somit die Bereitstellung eines Verfahrens zum Nachweis spezifischer Antikörper, bei dem die Nachteile des Standes der Technik mindestens teilweise beseitigt sind, und das insbesondere bei erst seit kurzem erfolgten Serokonversionen und neuen Mikroorganismen-Subtypen eine ausreichende Sensitivität besitzt. Weiterhin soll durch das erfindungsgemäße Verfahren eine Verringerung des Hook-Effekts erreicht werden.

Dieses Problem wird gelöst durch ein Verfahren zur immunologischen Bestimmung eines spezifischen Antikörpers in einer Probeflüssigkeit, wobei die Probeflüssigkeit in Gegenwart einer Festphase mit zwei gegen den zu bestimmenden Antikörper gerichteten Antigenen inkubiert, wobei das erste Antigen mindestens eine Markierungsgruppe trägt und das zweite Antigen (a) an die Festphase gebunden ist oder (b) in einer an die Festphase bindefähigen Form vorliegt, und den zu bestimmenden Antikörper durch Bestimmung der Markierungsgruppe in der Festphase oder/und in der flüssigen Phase nachweist, dadurch gekennzeichnet, daß mindestens eines der beiden Antigene mehrere Epitopbereiche umfaßt, die mit dem zu bestimmenden Antikörper reagieren.

Überraschenderweise wurde festgestellt, daß in Brückentest-Immunoassays unter Verwendung von mindestens einem multimeren Antigen, d.h. einem Antigen mit multiplen Epitopbereichen, die Sensitivität des Tests insbesondere gegenüber Seren mit Antikörpern verbessert wird, die gegenüber dem verwendeten Antigen eine geringe Affinität besitzen. Weiterhin führt das erfindungsgemäße Verfahren zu einer deutlichen Verringerung des Risikos falsch negativer Bewertungen von hochtitrigen Proben infolge des Hook-Effekts. Die Optimierung der Antigenpräsentation über die Erhöhung der Epitopdichte im Brückentestkonzept führt generell zu einer Verbesserung der Reaktivität mit spezifischen Immunglobulinen in polyklonalen Seren, wie sie in einer Probeflüssigkeit, wie z.B. Serum, vorliegen. Ein weiterer Vorteil des erfindungsgemäßen Verfahrens besteht darin, daß multimere Antigene gegenüber monomeren Antigenen eine deutlich verbesserte Stabilität aufweisen können.

Bei einem Verfahren zur immunologischen Bestimmung eines spezifischen Antikörpers nach dem Brückentestkonzept werden zwei Antigene verwendet. In einer ersten bevorzugten Ausführungsform des erfindungsgemäßen Verfahrens verwendet man als markiertes Antigen ein multimeres Antigen und als festphasenseitiges Antigen ein monomeres Antigen. In einer zweiten Ausführungsform des erfindungsgemäßen Verfahrens kann man als festphasenseitiges Antigen ein multimeres Antigen und als markiertes Antigen ein monomeres Antigen verwenden. In einer dritten bevorzugten Ausführungsform kann man als markiertes Antigen und als festphasenseitiges Antigen jeweils multimere Antigene verwenden.

Die multimeren Antigene enthalten multiple Epitopbereiche, d.h. immunologisch mit dem zu bestimmenden Antikörper reagierende Strukturen, vorzugsweise Peptid- oder Polypeptidsequenzen. Die Epitopbereiche sind vorzugsweise über immunologisch inaktive Bereiche, z.B. über Spacerbereiche, miteinander verknüpft. Vorzugsweise verwendet man multimere Antigene, die mehrere gleiche Epitopbereiche umfassen.

Die erfindungsgemäßen multimeren Antigene enthalten vorzugsweise von mehr als 1 bis 80 immunologisch reaktive Epitopbereiche, besonders bevorzugt von mehr als 1 bis 40 Epitopbereiche. Die Epitopbereiche können an einen hochmolekularen Träger gekoppelt sein oder direkt bzw. über Spacerbereiche miteinander verknüpft sein.

Die Epitopbereiche sind vorzugsweise immunologisch reaktive synthetische Peptidsequenzen mit einer Länge von 6 bis 50 Aminosäuren oder rekombinante Polypeptidsequenzen mit einer Länge von vorzugsweise bis zu 1000 Aminosäuren. Synthetische Peptidepitope enthalten neben dem eigentlichen Epitopbereich vorzugsweise noch einen Spacerbereich, der beispielsweise zur Kopplung mit anderen Epitopen oder einem Täger oder/und zur Kopplung von Markierungs- bzw. Festphasenbindegruppen dienen kann.

Der Spacerbereich ist vorzugsweise eine immunologisch inaktive Peptidsequenz mit einer Länge von 1 bis 10 Aminosäuren. Die Aminosäuren des Spacerbereichs werden vorzugsweise ausgewählt aus der Gruppe bestehend aus Glycin, β-Alanin, γ-Aminobuttersäure, ε-Aminocapronsäure, Lysin und Verbindungen der Strukturformel $NH_2[(CH_2)_nO]_x-CH_2-CH_2-COOH$, worin n 2 oder 3 ist und x 1 bis 10 ist.

Der Spacerbereich ist vorzugsweise eine kontinuierliche Abfolge von Aminosäuren am Aminoterminus oder/und Carboxyterminus des Epitopbereichs.

Bei einem Immunoassay nach dem Brückentestkonzept wird ein erstes markiertes Antigen verwendet. Für das erfindungsgemäße Verfahren können alle bekannten Markierungsgruppen verwendet werden, z.B. radioaktive und nicht-radioaktive Markierungsgruppen. Die bevorzugten nicht-radioaktiven Markierungsgruppen können direkt oder/und indirekt nachweisbar sein. Bei einer direkt nachweisbaren Markierung ist die ein nachweisbares Meßsignal erzeugende Gruppe direkt auf dem Antigen lokalisiert. Beispiele für solche direkt signalerzeugenden Gruppen sind Chromogene (fluoreszierende oder lumineszierende Gruppen, Farbstoffe), Enzyme, NMR-aktive Gruppen oder Metallpartikel, die auf bekannte Weise an ein Peptid- oder Polypeptidantigen gekoppelt sind. Vorzugsweise ist die direkt nachweisbare Markierungsgruppe ein durch Fluoreszenz oder Elektrochemolumineszenz nachweisbares Metallchelat, besonders bevorzugt Ruthenium-, Rhenium,- Iridium- oder Osmiumchelat, insbesondere ein Rutheniumchelat, z.B. ein Ruthenium- (bispyridyl) $_3{}^{2+}$-chelat. Weitere geeignete Metallchelat-Markierungsgruppen sind beispielsweise in EP-A-0 580 979, WO 90/05301, WO 90/11511 und WO 92/14138 beschrieben. Auf diese Dokumente wird hiermit Bezug genommen.

Eine andere Art der Markierung, die sich für die erfindungsgemäßen Antigene eignet, ist die indirekt nachweisbare Markierung. Bei dieser Art der Markierung ist das Antigen mit einer indirekt nachweisbaren Gruppe gekoppelt, z.B. einer Biotin- oder Haptengruppe, die wiederum durch Reaktion mit einem geeigneten Bindepartner (Streptavidin, Avidin, bzw. Anti-Hapten-Antikörper), der wiederum eine signalerzeugende Gruppe trägt, nachweisbar ist. Vorzugsweise wird als indirekte Markierungsgruppe als Hapten ein organisches Molekül mit einem Molekulargewicht von 100 bis 2000, vorzugsweise von 150 bis 1000 verwendet.

Die Haptene sind mit einem spezifischen Rezeptor für das jeweilige Hapten bindefähig. Beispiele für Rezeptoren sind Antikörper, Antikörperfragmente, die gegen das Hapten gerichtet sind, oder ein anderer spezifischer Bindepartner für das Hapten, z.B. Streptavidin oder Avidin, wenn das Hapten Biotin ist. Vorzugsweise wird das Hapten ausgewählt aus der Gruppe bestehend aus Sterinen, Gallensäuren, Sexualhormonen, Corticoiden, Cardenoliden, Cardenolid-Glycosiden, Bufadienoliden, Steroid-Sapogeninen und Steroidalkaloiden. Besonders bevorzugt wird das Hapten ausgewählt aus der Gruppe bestehend aus Cardenoliden und Cardenolid-Glycosiden. Vertreter dieser Stoffklassen sind Digoxigenin, Digitoxigenin, Gitoxigenin, Strophantidin, Digoxin, Digitoxin, Ditoxin und Strophantin, wobei Digoxigenin und Digoxin besonders bevorzugt sind. Ein anderes geeignetes Hapten ist beispielsweise Fluorescein oder ein geeignetes Fluoresceinderivat.

Der Rezeptor für das Hapten ist mit einer signalerzeugenden Gruppe, vorzugsweise einem Enzym, wie etwa Peroxidase, alkalische Phosphatase, ß-Galactosidase, Urease oder Q-ß-Replikase, gekoppelt. Die signalerzeugende Gruppe kann jedoch auch eine chromogene, radioaktive oder NMR-aktive Gruppe oder ein Metallpartikel (z.B. Gold) sein. Die Kopplung des Haptens mit dem Antigen kann beispielsweise dadurch erfolgen, daß das Hapten in Form eines Aktivesterderivats an den Aminoterminus oder/und an freie Aminoseitengruppen des Peptid- oder Polypeptidantigens gekoppelt wird.

Der Begriff "Aktivester" im Sinne der vorliegenden Erfindung umfaßt aktivierte Estergruppen, die mit freien Aminogruppen von Peptiden unter solchen Bedingungen reagieren können, daß keine störenden Nebenreaktionen mit anderen reaktiven Gruppen des Peptids auftreten können. Vorzugsweise wird als Aktivesterderivat ein N-Hydroxysuccinimidester verwendet. Beispiele für geeignete Hapten-Aktivesterderivate sind Digoxin-4'''-hemiglutarat-N-hydroxysuccinimidester, Digoxigenin-3-carboxymethylether-N-hydroxysuccinimidester, Digoxigenin-3-O-methylcarbonyl-ε-aminocapronsäure-N-hydroxysuccinimidester, Digoxigenin-3-hemisuccinat-N-hydroxysuccinimidester, Digitoxin-4'''-hemiglutarat-N-hydroxysuccinimidester und Digitoxigenin-3-hemisuccinat-N-hydroxysuccinimidester. Diese Haptenderivate sind von der Fa. Boehringer Mannheim GmbH (Mannheim, BRD) kommerziell erhältlich. Neben den N-Hydroxysuccinimidestern können auch analoge p-Nitrophenyl-, Pentafluorphenyl-, Imidazolyl- oder N-Hydroxybenzotriazolylester verwendet werden.

Neben dem ersten markierten Antigen wird bei dem erfindungsgemäßen Verfahren auch ein zweites Antigen verwendet, das an eine Festphase gebunden ist oder in einer an eine Festphase bindefähigen Form vorliegt, und ebenfalls ein multimeres Antigen sein kann. Die Bindung zwischen dem festphasenseitigen Antigen und der Festphase kann kovalent oder adsorptiv sein und direkt, über chemische Linkergruppen oder über eine spezifische Wechselwirkung, z.B. Biotin - Streptavidin/Avidin, Antigen - Antikörper, Kohlenhydrat - Lectin, erfolgen. Vorzugsweise ist das festphasenseitige Antigen ein biotinyliertes Antigen und die Festphase entsprechend mit Streptavidin oder Avidin beschichtet. Die Kopplung von Biotingruppen mit dem Antigen kann auf bekannte Weise, z.B. durch die Einführung von Biotin-Aktivesterderivaten erfolgen. Derartige Methoden sind dem Fachmann bekannt.

Die Anzahl von Markierungs- oder Festphasenbindegruppen auf dem multimeren Antigen ist variabel, d.h. es können eine oder mehrere Gruppen vorhanden sein. In manchen Ausführungsformen des erfindungsgemäßen Verfahrens ist es bevorzugt, wenn mindestens 3, und besonders bevorzugt 3 bis 20 Markierungs- oder Festphasenbindegruppen vorhanden sind. Auf diese Weise kann eine überraschend hohe Sensitivitätsverbesserung und eine signifikante Abnahme des Hook-Effekts (falsch negative Bewertung von stark positiven Proben) erreicht werden.

Die vorliegende Erfindung beruht auf der Erkenntnis, daß ein immunologischer Test zur Bestimmung eines spezifischen Antikörpers in einer Probeflüssigkeit vorteilhaft ist, wenn mindestens eines der für diesen Test verwendeten beiden Antigene ein multimeres Antigen ist, d.h. mehrere Epitopbereiche, vorzugsweise mehrere gleiche Epitopbereiche umfaßt. Der Begriff "Epitopbereich" im Sinne der vorliegenden Erfindung bedeutet eine Struktur, vorzugsweise eine Peptid- oder Polypeptidsequenz, die eine spezifische Reaktion mit dem zu bestimmenden Antikörper zeigt. Zur Anordnung mehrerer Epitopbereiche auf dem multiplen Antigen gibt es prinzipiell mehrere Möglichkeiten.

In einer ersten Ausführungsform verwendet man als multimeres Antigen einen nicht mit dem zu bestimmenden Antikörper reagierenden Träger, an den die Epitopbereiche kovalent gekoppelt sind. Beispiele für geeignete Träger sind Peptide, Polypeptide oder synthetische Träger, z.B. Dextrane, Goldpartikel, Dendriten etc. Beispiele für geeignete Polypeptide sind Albumine, z.B. Rinderserum-Albumin, unspezifische Immunglobuline, Immunglobulinfragmente, β-Galactosidase, Polylysin und andere Proteine mit symmetrischer Struktur. Bei der Verwendung des Trägers ist darauf zu achten, daß er keine Kreuzreaktivität mit Antikörpern in der Probeflüssigkeit zeigt.

Die Epitopbereiche sind vorzugsweise über einen bifunktionellen Linker an reaktive Gruppen des Trägers, z.B. $NH_2$-Gruppen oder SH-Gruppen, gekoppelt. Vorzugsweise erfolgt die Kopplung über $NH_2$-Gruppen des Trägers.

In dieser Ausführungsform der Erfindung verwendet man vorzugsweise ein Antigen der allgemeinen Formeln

$$(P-)_n T(-L)_m \quad (Ia)$$

oder

$$T(-P-L_m)_n \quad (Ib)$$

wobei T einen Träger bedeutet, P Peptid- oder Polypeptidsquenzen bedeutet, die gleiche oder verschiedene, immunologisch reaktive Epitopbereiche enthalten und kovalent an den Träger gekoppelt sind, und L Markierungsgruppen oder zur Bindung an eine Festphase fähige Gruppen bedeutet, die kovalent an den Träger bzw. an die Peptid- oder Polypeptidsequenzen gekoppelt sind, n eine Zahl von größer 1 bis 40 ist und m eine Zahl von 1 bis 10 ist. Die Symbole n und m müssen keine ganzen Zahlen bedeuten, da die Belegung des Trägers mit Epitopgruppen oder mit Markierungs- bzw. Festphasenbindegruppen in einem Reaktionsansatz statistisch erfolgen kann. Vorzugsweise ist n größer oder gleich 2.

Die an den Träger gekoppelten Peptid- oder Polypeptidsequenzen umfassen vorzugsweise synthetische Peptidsequenzen mit einer Länge von 6 bis 50 Aminosäuren oder rekombinante Polypeptidsequenzen mit einer Länge von vorzugsweise bis zu 1000 Aminosäuren.

Synthetische Peptidsequenzen können gegebenenfalls neben dem eigentlichen Epitopbereich noch einen Spacerbereich wie oben definiert enthalten, der beispielsweise zwischen Epitop und Träger oder/und zwischen Epitop und Markierungs- bzw. Festphasenbindegruppe angeordnet sein kann.

Die Peptid- oder Polypeptidepitope können über den N-Terminus, den C-Terminus, oder über reaktive Gruppen in der Seitenkette an den Träger gekoppelt werden. Eine Möglichkeit der Kopplung besteht darin, die Trägermoleküle durch Reaktion mit bekannten Linker-Substanzen (z.B. Maleinimidohexansäure, Maleinimidopropionsäure, Maleinimidobenzoesäure) an einer $NH_2$-Gruppe zu aktivieren und ein SH-aktiviertes Peptidderivat kovalent an den Träger zu koppeln. Die Markierungs- oder Festphasenbindegruppen werden üblicherweise in Form von Aktivestern an das Trägermolekül oder/und an die Epitopbereiche gekoppelt. Es sind aber auch andere Kopplungsmöglichkeiten z.B. über bifunktionelle Photolinker, denkbar.

Für die Synthese von multimeren Antigenen, welche die Epitope gekoppelt an einem inerten Träger enthalten, werden die entsprechenden Peptide vorzugsweise mit einer reaktiven MercaptoFunktion, z.B. durch Einführen eines zusätzlichen Cysteinrests hergestellt. Das Peptid kann dabei entweder N-terminal, C-terminal oder auch beliebig in der Sequenz mit einem Linker modifiziert sein. Für die Umsetzung zum multimeren Antigen kann beispielsweise ein Träger, der primäre Aminogruppen enthält, zuerst mit dem entsprechenden Aktivesterderivat der Markierungsgruppe und anschließend mit Maleinimidoalkylgruppen beladen werden. Dadurch werden die Aminogruppen der ε-Aminoseitenkette von Lysinresten im Träger teilweise mit der Markierungsgruppe (z.B. Digoxigenin oder Bipyridylruthenium) oder der Festphasenbindegruppe (z.B. Biotin) markiert und zum anderen Teil in Maleinimidgruppen umgewandelt.

In einem weiteren Schritt wird dann das Peptid oder die Peptidmischung mit den gewünschten Epitopbereichen über die reaktive Mercaptofunktion an den Maleinimid-modifizierten Träger gekoppelt. Wenn die Markierungsgruppe direkt auf dem Peptid lokalisiert ist, erfolgt die Synthese des multimeren Antigens analog, nur wird jetzt ein entsprechend markiertes, SH-aktiviertes Peptid mit dem Träger umgesetzt.

In einer weiteren Ausführungsform der Erfindung kann man ein multimeres Antigen verwenden, daß mehrere direkt oder über Spacerbereiche miteinander kovalent gekoppelte Epitopbereiche umfaßt. Vorzugsweise erfolgt die Verknüpfung der Epitope mindestens teilweise über trifunktionelle Linkermoleküle, so daß das Antigen mindestens eine Verzweigungsstelle und vorzugsweise 1 bis 7 Verzweigungsstellen umfaßt.

Vorzugsweise verwendet man in dieser Ausführungsform ein Antigen der allgemeinen Formel II:

$$P^1\{P^2[P^3(P^4)_t]_s\}_r \qquad (II)$$

wobei $P^1$, $P^2$, $P^3$ und $P^4$ Peptidsequenzen mit einer Länge von bis zu 50 Aminosäuren bedeuten, wobei mindestens 2 Peptidsequenzen gleiche oder verschiedene immunologisch reaktive Epitopbereiche enthalten, r 1 oder 2 ist, s eine ganze Zahl von 0 bis 4 ist und t eine ganze Zahl von 0 bis 8 ist, wobei das Antigen mindestens eine Verzweigungsstelle und mindestens eine Markierungsgruppe oder eine zur Bindung an eine Festphase fähige Gruppe enthält.

Das Antigen der Formel II bildet eine baumartige Struktur mit maximal 7 Verzweigungsstellen, wenn $P^1$, $P^2$, $P^3$ und $P^4$ lineare Peptidsequenzen sind, und enthält vorzugsweise zwei bis acht gleiche oder verschiedene, immunologisch reaktive Epitopbereiche. Die Epitopbereiche sind vorzugsweise nicht direkt, sondern über Spacerbereiche miteinander verknüpft. Die Spacerbereiche sind vorzugsweise immunologisch inaktive Peptidsequenzen mit einer Länge von 1 bis 10 Aminosäuren, wie oben definiert. Nicht alle Peptidsequenzen $P^1$, $P^2$, $P^3$ und $P^4$ müssen Epitopbereiche enthalten, sondern es sind auch Strukturen möglich, bei denen diese Sequenzen nur aus Spacerbereichen bestehen. Verzweigungen können durch Verwendung trifunktioneller Aminosäuren, z.B. Lysin oder Ornithin, in die Struktur eingebaut werden.

Weiterhin enthält das Antigen der allgemeinen Formel II mindestens eine Markierungs- oder Festphasenbindegruppe, wie oben definiert. Diese Gruppen können beispielsweise selektiv an die Enden oder/und an reaktive Seitenketten der Peptidsequenzen gekoppelt werden.

Noch eine weitere Ausführungsform der multimeren Antigene sind die sogenannten Mosaikproteine, d.h. rekombinante Fusions-Polypeptide, deren Aminosäuresequenz mehrere immunologisch reaktive Epitopbereiche enthält, die gegebenenfalls über immunologisch inaktive Spacerbereiche verknüpft sind. Die rekombinanten Mosaikproteine sind erhältlich, indem man eine für das gewünschte Protein kodierende DNA-Sequenz herstellt und in einer rekombinanten Wirtszelle zur Expression bringt. Derartige Verfahren sind dem Fachmann auf dem Gebiet der Molekularbiologie bekannt und in Standardlehrbüchern (z.B. Sambrook et al., Molecular Cloning. A Laboratory Manual, 2nd Edition (1989), Cold Spring Harbor Laboratory Press) beschrieben. Die Einführung von Markierungs- oder Festphasenbindegruppen in das rekombinante Protein kann ebenfalls nach bekannten Methoden erfolgen.

In einer bevorzugten Ausführungsform der Erfindung sind die Epitopbereiche synthetische Peptidsequenzen mit einer Länge von 6 bis maximal 50, besonders bevorzugt bis maximal 30 Aminosäuren. In derartige Epitopbereiche können Markierungsgruppen bzw. Festphasenbindegruppen selektiv sowohl bezüglich ihrer Lokalisierung als auch bezüglich ihrer Anzahl eingeführt werden. Bei der synthetischen Herstellung besteht nämlich die Möglichkeit, durch Verwendung bestimmter Schutzgruppen an reaktiven Seitengruppen, z.B. primären Aminogruppen der eingesetzten Aminosäurederivate, diejenigen Positionen des Peptids gezielt auszuwählen, die nach selektiver Schutzgruppenabspaltung zur Reaktion mit der eingeführten Markierungsgruppe zur Verfügung stehen.

Hierzu wird das Peptid mit der gewünschten Aminosäuresequenz an einer Festphase, vorzugsweise mit einem kommerziellen Peptid-Synthesegerät (z..B. die Geräte A 431 oder A 433 von Applied Biosystems) hergestellt. Die Synthese erfolgt nach bekannten Methoden, vorzugsweise ausgehend vom Carboxylterminus des Peptids unter Verwendung von Aminosäurederivaten. Vorzugsweise werden Aminosäurederivate eingesetzt, deren für die Kupplung benötigte Amino-Endgruppe mit einem Fluorenylmethyloxycarbonyl (Fmoc)-Rest derivatisiert ist. Reaktive Seitengruppen der eingesetzten Aminosäuren enthalten Schutzgruppen, die nach Beendigung der Peptidsynthese ohne weiteres abspaltbar sind. Bevorzugte Beispiele hierfür sind Schutzgruppen, wie etwa Triphenylmethyl (Trt), t-Butylether (tBu), t-Butylester(0 tBu), tert.-Butoxycarbonyl (Boc) oder 2,2,5,7,8-Pentamethylchroman-6-sulfonyl (Pmc).

Die Aminoseitenketten von Lysinresten oder anderen Aminosäurederivaten mit primären Aminoseitengruppen, die sich an Positionen des Peptids befinden, die später mit einem Hapten, z.B. Digoxin oder Digoxigenin, derivatisiert werden sollen, sind mit einer ersten Aminoschutzgruppe versehen, die so ausgewählt wird, daß sie quantitativ unter bestimmten Reaktionsbedingungen, z.B. in Anwesenheit von Säure, abspaltbar sind. Ein Beispiel für eine geeignete säurelabile Schutzgruppe ist.Boc. Die Seitengruppen von Lysinresten oder anderen Aminosäureresten mit primären Aminoseitengruppen, an denen keine Kopplung eines Haptens gewünscht wird, sind mit einer zweiten Aminoschutzgruppe versehen, die so ausgewählt wird, daß sie unter den Bedingungen, bei denen die erste Schutzgruppe abspaltbar ist, selbst nicht abgespalten wird. Vorzugsweise ist die zweite Schutzgruppe auch unter denjenigen Bedingungen stabil, bei denen die Abspaltung des Peptids von der Festphase und die Abspaltung aller deren Schutzgruppen erfolgt. Beispiele für solche zweiten Schutzgruppen sind säurestabile Schutzgruppen, wie etwa Phenylacetyl. Neben den 20 natürlichen Aminosäuren kann das Peptid auch artefizielle Aminosäuren, wie etwa ßAlanin, γ-Aminobuttersäure, ε-Aminocapronsäure oder Norleucin enthalten. Diese artefiziellen Aminosäuren werden analog wie die natürlichen Aminosäuren für die Synthese in geschützter Form eingesetzt.

Nach Beendigung der Synthese erfolgt gegebenenfalls nach Freisetzung des Peptids von der Festphase eine Abspaltung von Schutzgruppen einschließlich der ersten Aminoschutzgruppen, die sich an den Positionen befinden, an denen die Kopplung des Haptens stattfinden soll. Dann wird das auf diese Weise erhaltene Produkt gereinigt, vorzugsweise durch HPLC. Anschließend erfolgt die Einführung der Hapten-Markierung durch Umsetzung des Peptids mit dem jeweils gewünschten Hapten-Aktivesterderivat, das mit freien primären Aminogruppen, d.h. mit der Amino-Endgruppe oder/und Aminoseitengruppen des Peptids, reagiert. Pro freie primäre Aminogruppe werden vorzugsweise 1,5 bis 2,5 Äquivalente Aktivester eingesetzt. Anschließend wird das Reaktionsprodukt aufgereinigt, vorzugsweise durch HPLC.

Enthält das Peptid noch Aminogruppen, die mit einer zweiten Schutzgruppe, wie etwa Phenylacetyl, derivatisiert sind, so werden diese Schutzgruppen im letzten Schritt entfernt. Die Entfernung von Penylacetylschutzgruppen kann beispielsweise enzymatisch mit immobilisierter oder löslicher Penicillin-G-Amidase in wäßriger Lösung mit organischem Solvensanteil bei Raumtemperatur erfolgen.

Enthalten die durch das erfindungsgemäße Verfahren hergestellten Peptide eine intramolekulare Disulfidbrükke, so kann die Peptidsequenz nach Beendigung der Synthese, aber vor Abspaltung der N-terminalen Fmoc-Schutzgruppe der letzten Aminosäure z.B. mit Jod in Hexafluorisopropanol/Dichlormethan (Cober et al. The Peptide Academic Press, New York, 1981, Seiten 145 bis 147) an der Festphase oxidiert, und anschließend die N-terminale Fmoc-Schutzgruppe abgespalten werden.

Die Einführung einer reaktiven SH-Gruppe kann beispielsweise durch Kupplung eines Cysteinrests an den Aminoterminus des Peptids erfolgen.

Die Einführung von Metallchelat-Markierungsgruppen in synthetische Peptide erfolgt (a) nach Synthese der gewünschten Peptidsequenz und vorzugsweise vor der Abspaltung des Peptids von der Festphase und vor der Abspaltung von Schutzgruppen an reaktiven Seitengruppen der zur Peptidsynthese verwendeten Aminosäurederivate durch Kopplung eines aktivierten lumineszierenden Metallchelats, z.B. eines Aktivesterderivats, an die N-terminale primäre Aminogruppe des Peptids oder/und (b) während der Synthese des Peptids durch Einführung von Aminosäurederivaten, die kovalent mit einer lumineszierenden Metallchelat-Markierungsgruppe gekoppelt sind, z.B. über ein ε-derivatisiertes Lysin.

Die Synthese von verzweigten multimeren Antigenen kann dadurch erfolgen, daß eine durch zwei Fmoc-Gruppen geschützte Diaminocarbonsäure, wie etwa Lysin, eingesetzt wird. Die Biotinylierung der Peptide kann beispielsweise durch Einführung eines Biotinderivats an den N-Terminus erfolgen, während das Peptid noch an die Festphase gekoppelt ist.

Für das erfindungsgemäße Verfahren werden vorzugsweise Peptid- oder Polypeptidepitope aus pathogenen Organismen, z.B. Bakterien, Viren und Protozoen oder aus Autoimmun-Antigenen verwendet. Vorzugsweise stammt der immunologisch reaktive Epitopbereich aus viralen Antigenen, z.B. den Aminosäuresequenzen von HIV I, HIV II, HIV Subtyp O oder Hepatitis C-Virus (HCV).

Vorzugsweise werden HIV I-, HIV II- bzw. HIV-Subtyp O-Epitope aus den Regionen gp32, gp41, gp120 und gp24 ausgewählt. HCV-Epitope werden vorzugsweise aus der Core/Env-Region oder den Nicht-Strukturprotein-Regionen NS3, NS4 oder NS5 ausgewählt.

Besonders bevorzugt wird der Epitopbereich von HIV I, HIV II- oder HIV Subtyp O-Aminosäuresequenzen ausgewählt aus der Gruppe der Aminosäuresequenzen:

| | | | |
|---|---|---|---|
| NNTRKSISIG | PGRAFYT | | (I) |
| NTTRSISIGP | GRAFYT | | (II) |
| IDIQEERRMR | IGPGMAWYS | | (III) |
| QARILAVERY | LKDQQLLGIW | GASG | (IV) |
| LGIWGCSGKL | ICTTAVPWNA | SWS | (V) |
| KDQQLLGIWG | SSGKL | | (VI) |
| ALETLLQNQQ | LLSLW | | (VII) |
| LSLWGCKGKL | VCYTS | | (VIII) |
| WGIRQLRARL | LALETLLQN | | (IX) und |
| QAQLNSWGCA | FRQVCHTTVP | WPNDSLT | (X) |

oder Teilsequenzen davon, die eine Länge von mindestens 6 und vorzugsweise mindestens 8 Aminosäuren aufweisen.

Die Aminosäurensequenzen I bis III stammen aus der gp120-Region von HIV I, die Aminosäursquenzen IV bis IX stammen aus der gp41-Region von HIV I und die Aminosäuresequenz X stammt aus der gp32-Region von HIV II. Die Aminosäuresequenzen I bis X sind weiterhin in den Sequenzprotokollen SEQ ID NO. 1 bis SEQ ID NO. 10 dargestellt. Die Sequenzen V, VIII und X enthalten jeweils 2 Cysteinreste, die vorzugsweise in Form einer Disulfidbrücke vorliegen.

Der Epitopbereich von HCV-Aminosäuresequenzen wird vorzugsweise ausgewählt aus der Gruppe der Aminosäuresequenzen:

| | | | |
|---|---|---|---|
| SRRFAQALPV | WARPD | | (XI) |
| PQDVKFPGGG | QIVGGV | | (XII) |
| EEASQHLPYI | EQ | | (XIII) |
| QKALGLLQT | | | (XIV) |
| SRGNHVSPTH | YVPESDAA | | (XV) |
| PQRKNKRNTN | RRPQDVKFPG | | |
| GGQIVGW | | | (XVI) und |
| AWYELTPAET | TVRLRAYMNT | PGLPV | (XVII) |

oder Teilsequenzen davon, die eine Länge von mindestens 6 und vorzugsweise mindestens 8 Aminosäuren aufweisen. Die Sequenz XI stammt aus der NS5-Region, die Sequenzen XII und XVI aus der Core Region, die Sequenzen XIII, XIV und XV aus der NS4 Region und die Sequenz XVII aus der NS3-Region von HCV. Die Aminosäuresequenzen XI bis XVII sind weiterhin in den Sequenzprotokollen SEQ ID NO. 11 bis SEQ ID NO. 17 dargestellt.

Ein weiterer Gegenstand der vorliegenden Erfindung ist ein Reagenz zur immunologischen Bestimmung eines spezifischen Antikörpers in einer Probeflüssigkeit umfassend eine reaktive Festphase, zwei gegen den zu bestimmenden Antikörper gerichtete Antigene, wobei das erste Antigen eine Markierungsgruppe trägt und das zweite Antigen (a) an die Festphase gebunden ist oder (b)in einer an die Festphase bindefähigen Form vorliegt, dadurch gekennzeichnet, daß mindestens eines der beiden Antigene mehrere Epitopbereiche umfaßt, die mit dem zu bestimmenden Antikörper reagieren.

In einer Ausführungsform der vorliegenden Erfindung enthält das Reagenz ein erstes markiertes Antigen mit mehreren Epitopbereichen, das mindestens eine Hapten-Markierungsgruppe trägt und einen Rezeptor für das Hapten, der wiederum eine signalerzeugende Gruppe enthält. Weiterhin bevorzugt ist ein Reagenz umfassend ein zweites festphasenseitiges Antigen mit mehreren Epitopbereichen, das mindestens eine Biotingruppe trägt, und eine mit Streptavidin oder Avidin beschichtete reaktive Festphase.

Noch ein weiterer Gegenstand der vorliegenden Erfindung ist die Verwendung von multimeren Antigenen, die mehrere immunologisch reaktive Epitopbereiche umfassen, in einem immunologischen Testverfahren zur Bestimmung von spezifischen Antikörpern in einer Probeflüssigkeit.

Vorzugsweise werden solche Antikörper bestimmt, die auf eine Infektion durch Mikroorganismen, wie etwa Bakterien, Viren oder Protozoen, hinweisen. Besonders bevorzugt werden gegen Viren gerichtete Antikörper, z.B. gegen HIV oder Hepatitis-Viren gerichtete Antikörper bestimmt. Die Probeflüssigkeit ist vorzugsweise Serum oder Plasma, besonders bevorzugt humanes Serum oder Plasma. Weiterhin ist bevorzugt, daß die erfindungsgemäßen multimeren Antigene bei einem immunologischen Verfahren im Brückentestformat eingesetzt werden.

Die Testdurchführung beinhaltet vorzugsweise ein Mischen der Probeflüssigkeit mit dem ersten Antigen und dem festphasenseitigen zweiten Antigen, um einen markierten, immobilisierten Komplex aus erstem Antigen, Antikörper und festphasengebundenem zweiten Antigen zu erhalten. Gegenüber anderen Testformaten zum Nachweis von Antikörpern führt das Brückentestformat sowohl zu einer Verbesserung der Sensitivität, d.h. es werden alle Immunglobulinklassen, wie etwa IgG, IgM, IgA und IgE, erkannt, als auch der Spezifität, d.h. es wird die unspezifische Reaktivität verringert. Durch das erfindungsgemäße Verfahren gelingt somit ein klassenunabhängiger Nachweis von spezifischem Immunglobulin mit verbesserter Sensitivität. Die Sensivitätsverbesserung betrifft insbesondere die zusätzliche Erkennung von niedrigaffinen Immunglobulinen, z.B. IgM mit multimeren Antigenen, wohingegen mit monomeren Antigenen nur hochaffine Antikörper gut erkannt werden. Die Spezifität und Sensitivität des Doppelantigen-Brückentests kann weiterhin verbessert werden, wenn man eine Zweischritt-Testführung verwendet, bei der in einem ersten Schritt die Probeflüssigkeit mit dem ersten und dem zweiten Antigen vermischt, und anschließend, vorzugsweise nach 1 bis 4 h, besonders bevorzugt nach 1,5 bis 2,5 h, der Rezeptor für die Haptenmarkierung des ersten Antigens, der die signalerzeugende Gruppe trägt, zugegeben wird.

Die Erfindung betrifft schließlich auch neue Antigene der Formeln (Ia), (Ib) und (II) wie oben definiert.

Weiterhin wird die vorliegende Erfindung durch die nachfolgenden Beispiele, Sequenzprotokolle und Figuren beschrieben.

Es zeigen

| | |
|---|---|
| SEQ ID NO. 1: | die Aminosäuresequenz eines Epitops aus dem gp120-Bereich von HIV I; |
| SEQ ID NO. 2: | die Aminosäuresequenz eines weiteren Epitops aus dem gp120-Bereich von HIV I; |
| SEQ ID NO. 3: | die Aminosäuresequenz eines weiteren Epitops aus dem gp120-Bereich von HIV I, Subtyp O; |
| SEQ ID NO. 4: | die Aminosäuresequenz des Epitops aus dem gp41-Bereich von HIV I; |
| SEQ ID NO. 5: | die Aminosäuresequenz eines weiteren Epitops aus dem gp41-Bereich von HIV I; |
| SEQ ID NO. 6: | die Aminosäuresequenz noch eines weiteren Epitops aus dem gp41-Bereich von HIV I; |
| SEQ ID NO. 7: | die Aminosäuresequenz eines Epitops aus dem gp41-Bereich von HIV I, Subtyp O; |
| SEQ ID NO. 8: | die Aminosäuresequenz eines weiteren Epitops aus dem gp41-Bereich von HIV I, Subtyp O; |
| SEQ ID NO. 9: | die Aminosäuresequenz noch eines weiteren Epitops aus dem gp41-Bereich von HIV I, Subtyp O; |
| SEQ ID NO.10: | die Aminosäuresequenz eines Epitops aus dem gp32-Bereich von HIV II; |
| SEQ ID N0.11: | die Aminosäuresequenz eines Epitops aus dem NS5-Bereich von HCV; |
| SEQ ID NO.12: | die Aminosäuresequenz eines Epitops aus dem Core-Bereich von HCV; |
| SEQ ID NO.13: | die Aminosäuresequenz eines Epitops aus dem NS4-Bereich von HCV; |
| SEQ ID NO.14: | die Aminosäuresequenz eines weiteren Epitops aus dem NS4-Bereich von HCV; |
| SEQ ID NO.15: | die Aminosäuresequenz noch eines weiteren Epitops aus dem NS4-Bereich von HCV; |
| SEQ ID N0.16: | die Aminosäuresequenz eines weiteren Epitops aus dem Core-Bereich von HCV; |
| SEQ ID NO.17: | die Aminosäuresequenz eines Epitops aus dem NS3-Bereich von HCV; |
| Figur 1: | die Aminosäuresequenz des rekombinanten HIV p24 Antigens, |
| Figur 2: | einen Vergleich der Meßsignale in einem Doppel-antigen-Brückentest bei Verwendung eines monomeren und eines multimeren ruthenylierten HIV-gp120 Antigens, und |
| Figur 3: | einen Vergleich der Meßsignale in einem Doppel-antigen-Brückentest bei Verwendung eines monomeren und eines multimeren biotinylierten HIV-gp41-Antigens. |

Beispiel 1

Herstellung von Peptid-Epitopbereichen

Die Peptid-Epitopbereiche wurden mittels Fluorenylmethyloxycarbonyl- (Fmoc) -Festphasenpeptidsynthese an einem Batch-Peptidsynthesizer, z.B. von Applied Biosystems A431 oder A433, hergestellt. Dazu wurden jeweils 4.0 Äquivalente der in Tabelle 1 dargestellten Aminosäurederivate verwendet:

Tabelle 1:

| | |
|---|---|
| A | Fmoc-Ala-OH |
| C | Fmoc-Cys(Trt)-OH |
| D | Fmoc-Asp(OtBu)-OH |
| E | Fmoc-Glu (OtBu) -OH |
| F | Fmoc-Phe-OH |
| G | Fmoc-Gly-OH |
| H | Fmoc-His(Trt)-OH |
| I | Fmoc-Ile-OH |
| K1 | Fmoc-Lys(Phenylacetyl)-OH |
| K2 | Fmoc-Lys(Boc)-OH |
| K3 | Boc-Lys(Fmoc)-OH |
| K4 | Fmoc-Lys(BPRu)-OH |
| L | Fmoc-Leu-OH |
| M | Fmoc-Met-OH |
| N | Fmoc-Asn(Trt)-OH |
| P | Fmoc-Pro-OH |
| Q | Fmoc-Gln(Trt)-OH |
| R | Fmoc-Arg(Pmc)-OH |
| S | Fmoc-Ser(tBu)-OH |
| T | Fmoc-Thr(tBu)-OH |
| U | Fmoc-ßAlanin-OH |
| V | Fmoc-Val-OH |
| W | Fmoc-Trp-OH |
| Y | Fmoc-Tyr(tBu)-OH |
| Z | Fmoc-ε-Aminocapronsäure-OH |
| Nle | Fmoc-Norleucin-OH |
| Abu | Fmoc-γ-Aminobuttersäure-OH |

Bei Anwesenheit von Cysteinresten in der Peptidsequenz erfolgte unmittelbar nach Beendigung der Synthese eine Oxidation an der Festphase mit Jod in Hexafluorisopropanol/Dichlormethan.

Die Aminosäuren oder Aminosäurederivate wurden in N-Methylpyrrolidon gelöst. Das Peptid wurde an 400-500 mg 4-(2',4'-Dimethoxyphenyl-Fmoc-Aminomethyl)-Phenoxy-Harz (Tetrahedron Letters 28 (1987), 2107) mit einer Beladung von 0,4-0,7 mmol/g aufgebaut (JACS 95 (1973), 1328). Die Kupplungsreaktionen wurden bezüglich des Fmoc-Aminosäurederivats mit 4 Äquivalenten Dicyclohexylcarbodiimid und 4 Äquivalenten N-Hydroxybenzotriazol in Dimethylformamid als Reaktionsmedium während 20 min durchgeführt. Nach jedem Syntheseschritt wurde die Fmoc-Gruppe mit 20%igem Piperidin in Dimethylformamid in 20 min abgespalten.

Die Freisetzung des Peptids vom Syntheseharz und die Abspaltung der säurelabilen Schutzgruppen - mit Ausnahme der Phenylacetylschutzgruppe - erfolgte mit 20 ml Trifluoressigsäure, 0,5 ml Ethandithiol, 1 ml Thioanisol, 1,5 g Phenol und 1 ml Wasser in 40 min bei Raumtemperatur. Die Reaktionslösung wurde anschlies- send mit 300 ml gekühltem Diisopropylether versetzt und zur vollständigen Fällung des Peptids 40 min bei 0°C gehalten. Der Niederschlag wurde abfiltriert, mit Diisopropylether nachgewaschen, mit wenig 50 %-iger Essigsäure gelöst und lyophilisiert. Das erhaltene Rohmaterial wurde mittels präparativer HPLC an Delta-PAK RP C18-Material (Säule 50 x 300 mm, 100 Å, 15 $\mu$) über einen entsprechenden Gradienten (Eluent A: Wasser, 0,1% Trifluoressigsäure, Eluent B: Acetonitril, 0,1% Trifluoressigsäure) in ca. 120 min. aufgereinigt. Die Identität des eluierte Materials wurde mittels Ionenspray-Massenspektrometrie geprüft.

Die Einführung einer Hapten-, z.B. einer Digoxigenin- bzw. Digoxin-Markierung erfolgte über Kopplung der entsprechenden Aktivester-Derivate, z.B. Digoxigenin-3-carboxymethylether-N-hydroxysuccinimidester (Boehringer Mannheim GmbH, Mannheim, BRD) an die freien Aminogruppen des Peptids in Lösung. Das zu derivatisierende Peptid wurde in einer Mischung aus DMSO und 0,1 M Kaliumphosphat-Puffer pH 8,5 gelöst. Anschließend wurden 2 Äquivalente Aktivester pro freie primäre Aminofunktion in wenig DMSO gelöst zugetropft und bei Raumtemperatur gerührt.

Der Umsatz wurde über analytische HPLC verfolgt. Das Produkt wird mittels präparativer HPLC aufgereinigt.

Das Lysin-Derivat K1 wurde für Positionen verwendet, an denen keine Haptenmarkierung stattfinden sollte. Das Lysin-Derivat K2 wurde für Positionen verwendet, an denen eine Haptenmarkierung stattfinden sollte. Das Lysin-Derivat K3 wurde zur Kopplung der ε-Aminogruppe an das Peptid im Spacerbereich verwendet.

Enthielt das Peptid noch mit Phenyacetyl geschützte Lysine, so wurde diese Schutzgruppe im letzten Schritt enzymatisch mit Penicillin-G-Amidase in wäßrigem Milieu mit organischem Solvens-Anteil bei Raumtemperatur abgespalten. Das Enzym wurde abfiltriert und das Peptid über präparative HPLC aufgereinigt. Die Identität des eluierten Materials wurde mittels Ionenspray-Massenspektrometrie geprüft.

Die Einführung einer Ruthenium-Markierungsgruppe erfolgte entweder N-terminal über ein Ruthenium (bispyridyl)$_3$ -Carbonsäurederivat (BPR$_U$-C00H), z.B. Ru-(bispyridyl)32+-N_hydroxysuccinimidester oder in der Sequenz über einen ∈-derivatisierten Lysinrest K4 (Fmoc-Lys (BPRu) OH) .

Die Einführung einer Biotinmarkierung erfolgte entweder N-terminal durch eine Derivatisierung am Harz (Biotin-Aktivester) oder innerhalb der Sequenz analog der Einführung einer Ruthenium-Markierung über ein entsprechend mit Biotin ∈ derivatisiertes Lysin.

Die Synthese verzweigter multimerer Peptide erfolgte analog der Synthese der linearen Peptide. Als Festphase wurde hier ein niedriger beladenes Harz, z.B. mit einer Beladung von 0,2 mmol/g gewählt. Für die Verzweigung wurde eine bis-Fmocgeschützte Diaminocarbonsäure, wie etwa Fmoc-Lys(Fmoc)-OH eingesetzt.

In den Tabellen 2 und 3 findet sich eine Auflistung von hergestellten Peptiden.

Aus den Bereichen gp120, gp41 und gp32 von HIV I bzw. HIV II wurden die in Tabelle 2a-2d dargestellten Peptidverbindungen hergestellt.

Tabelle 2a: SH-aktivierte lineare Peptide

| | |
|---|---|
| gp41/1 | CUZU-WGIRQLRARLLALETLLQN |
| gp41/2 | CUZU-LSLWGCKGKLVCYTS |
| gp41/4 | CUZU-ALETLLQNQLLSLW |
| gp120 | CUZU-IDIQEMRIGPMAWYS |

Tabelle 2b: Digoxigenin-markierte lineare Peptide

| | |
|---|---|
| gp120 | Digoxigenin-3-cme-UZU-NNTRKSISIGPGRAFYT<br>Digoxigenin-3-cme-UZ-NTTRSISIGPGRAFY<br>Digoxigenin-3-cme-UZU-IDIQEERRMRIGPGMAWYS |
| gp41/1 | Digoxigenin-3-cme-UZU-AVERYLKDQQLLGIW<br>Digoxigenin-3-cme-ZUZU-AVERYLKDQQLLGIW<br>Digoxigenin-3-cme-UZ-QARILAVERYLKDQQLLGIWGASG<br>Digoxigenin-3-cme-ZGGGG-QARILAVERYLKDQQLLGIWGASG<br>Digoxigenin-3-cme-UZU-WGIRQLRARLLALETLLQN |
| gp41/2 | Digoxigenin-3-cme-UZU-LGIWGCSGKLICTTAV<br>LGIWGCSGK-(cme-3-Digoxigenin)-LICTTAV |

(fortgesetzt)

| | |
|---|---|
| | Digoxigenin-3-cme-UZU-LGIWGCSGK-(cme-3-Digoxigenin)-LICTTAV<br>Digoxigenin-3-cme-ZU-GCSGKLICTTAVPWNASWS<br>GCSGK-(cme-3-Digoxigenin)-LICTTAVPWNASWS<br>GCSGKLICTTAVPWNASWSK(cme-3-Digoxigenin)G<br>Digoxigenin-3-cme-UZU-LSLWGCKGKLVCYTS |
| gp41/3 | Digoxigenin-3-cme-UZU-KDQQLLGIWGSSGKL |
| gp41/4 | Digoxigenin-3-cme-UZU-ALETLLQNQLLSLW |
| gp32 | Digoxigenin-3-cme-Z-NSWGCAFRQVCHTT |

Tabelle 2c: Ruthenylierte lineare Peptide

| | |
|---|---|
| gp120 | BPRu-UZU-NNTRKSISIGPGRAFYT<br>BPRu-UZ-NTTRSISIGPGRAFY<br>BPRu(ethylenglykol)-UZ-NTTRSISIGPGRAFY<br>NNTRKSISIGPGRAFYT-K(BPRu)<br>BPRu-UZU-IDIQEERRMRIGPGMAWYS |
| gp41/1 | BPRu-UZU-AVERYLKDQQLLGIW<br>BPRu-UGGG-QARILAVERYLKDQQLLGIWGASG<br>BPRu-GGGG-QARILAVERYLKDQQLLGIWGASG<br>BPRu-UZU-WGIRQLRARLLALETLLQN |
| gp41/2 | BPRu-UZU-LGIWGCSGKLICTTAV<br>BPRu-UGGG-GCSGKLICTTAVPWNASWS (GCSGKLICTTAVPWNASWS)K-(BPRu) |
| gp41/3 | BPRu-UZU-KDQQLLGIWGSSGKL |
| gp41/4 | BPRu-UZU-ALETLLQNQLLSLW |
| gp32 | BPRu-UZU-NSWGCAFRQVCHTT<br>BPRu-GGG-QAQLNSWGCAFRQVCHTTVPWPNDSLT |

Tabelle 2d: Verzweigte Peptide

| | |
|---|---|
| gp120 | (NTTRSISIGPGRAFY-AbuZ AbuZ)$_2$-K-Z-AbuZ-K-(Bi)<br>((NTTRSISIGPGRAFY-ZU)$_2$-K-UU-K-(Bi)<br>((NNTRKSISIGPGRAFYT-UZU-K)$_2$-UZU-<br>NNTRKSISIGPGRAFYT-UZU-K)$_2$-UZU-Bi |
| gp120 | (NTTRSISIGPGRAFY-ZU)$_2$-K-UU-K-(BPRu) |

Aus dem NS5-Bereich, dem NS4-Bereich, dem Core-Bereich und dem NS3-Bereich von HCV wurden die in den folgenden Tabellen 3a-d dargestellten Peptide synthetisiert.

Tabelle 3a: SH-aktivierte lineare Peptide

| | |
|---|---|
| NS4/3 | C-UZ-SRGNHVSPTHYVPESDAA |

Tabelle 3b: Hapten-markierte lineare Peptide

| | |
|---|---|
| NS5/1 | Digoxigenin-3-cme-UZU-SRRFAQALPVWARPD |
| Core2m | Digoxigenin-3-cme-U-PQDVKFPGGGQIVGGV |
| NS4/1 | Digoxigenin-3-cme-UU-Nle-EEASQHLPYIEQ |

(fortgesetzt)

| | |
|---|---|
| NS4/2 | Digoxigenin-3-cme-UU-QKALGLLQT |
| NS4/3 | Digoxigenin-3-cme-UZU-SRGNHVSPTHYVPESDAA |
| Corel | Digoxigenin-3-cme-UZU-KNKRNTNRR |
| Corel+2 | Digoxigenin-3-cme-U-PQRKNKRNTNRRPQDVKFPGGGQIVGVV |
| NS 3/1 | Digoxigenin-3-cme-UZ-AWYELTPAETTVRLRAYMNTPGLPV |

Tabelle 3c: Ruthenylierte lineare Peptide

| | |
|---|---|
| Core1 | BPRu-GGGG-KNKRNTNRR |
| Core1+2 | BPRu-UZU-KNKRNTNRRPQDVKFPGGGQIVGGV |
| NS4/1+2 | BPRu-UZ-SQHLPYIEQG-NleNle-LAEQFKQQALGLLQT |
| NS4/3m | BPRu-UZ-SRGNHVSPTHYVPESDAA |
| NS5/1 | BPRu-UZ-SRRFAQALPVWARPD |
| Core1+2+3 | BPRu-UZ-KNKRNTNRRPQDVKFPGGGQIVGGVLLPRR |
| Core1m | BPRu-UZ-NPKPQKKNKRNTNRR |
| Core3m | BPRu-UZ-GQIVGGVYLLPRRGPRLG |
| Core2m | BPRu-UZ-PQDVKFPGGGQIVGGV |
| NS4/3m-I | BPRu-UZU-SRGNHVSPTHYVPESDAA |
| NS4/1 | BPRu-UZU-SQHLPYIEQ |

Tabelle 3d: Verzweigte Peptide

| | |
|---|---|
| NS4/3m | $(SRGNHVSPTHYVPESDAA-UU)_2$ KUUK (BPRu)<br>$(SRGNHVSPTHYVPESDAA-UU)_4$ $K_2$KUUK (BPRu)<br>$(SRGNHVSPTHYVPESDAA-UU)_8$ $K_4U_4K_2$KUUK (BPRu)<br>$(SRGNHVSPTHYVPESDAA-UU)_2$ KUUK (Z-Bi)<br>$(SRGNHVSPTHY-VPESDAA-UU)_4$ $K_2$KUUK (Z-Bi)<br>$(SRGNHVSPTHYVPESDAA-UU)_8$ $K_4U_4K_2$ KUUK (Z-Bi) |

Beispiel 2

Herstellung von trägergebundenen multimeren Antigenen (Polyhaptenen) mit Peptidepitopen

Die entsprechenden Peptide wurden mit einer reaktiven Mercaptofunktion, z.B. durch Einführen von einem zusätzlichen Cystein hergestellt (vgl. Tabellen 2a und 2b). Das Peptid kann dabei entweder N-, C-terminal oder auch beliebig in der Sequenz mit einem sogenannten Linker modifiziert sein. Die Synthese der entsprechenden Peptide erfolgte wie in Beispiel 1 beschrieben.

Für die Umsetzung zum Polyhapten wurde der $NH_2$-Gruppen-haltige Träger zuerst mit dem entsprechenden Aktivester der Markierungsgruppen und anschließend mit Maleinimidoalkyl-Gruppen, durch Behandlung vorzugsweise mit Maleinimidohexyl- (MHS) oder Maleinimidopropyl-N-hydroxysuccinimidester (MPS), beladen. Dadurch wurden die primären Aminogruppen im Träger (z.B. ε-Aminoseitenkette von Lysinresten) teilweise markiert und zum anderen Teil in Maleinimidgruppen umgewandelt.

Die Umsetzung des Trägers mit den Aktivestern erfolgte vorzugsweise in 0,1 mol/l Kaliumphosphatpuffer pH 7,0-8,5 und einer Konzentration von 5-20 mg/ml innerhalb von 2-4 h bei Raumtemperatur. Die niedermolekularen Bestandteile wurden entweder über Dialyse oder Gelchromatographie (AcA 202-Gel, Eluent 0,1 mol/l Kaliumphosphatpuffer pH 7-8,5) abgetrennt.

In einem weiteren Schritt wurde dann das Peptid oder die Peptidmischung mit der reaktiven Mercaptofunktion an den MHSmodifizierten, gelabelten Träger in 0,1 mol/1 Kaliumphosphatpuffer pH 8,5 innerhalb von 6 h Raumtemperatur gekuppelt. Nicht umgesetztes Peptid wurde entweder mittels Dialyse oder Gelchromatographie abgetrennt.

Soll die Markierung direkt am Peptid sitzen, wurde das Polyhapten analog synthetisiert und ein entsprechend markiertes, SH-aktiviertes Peptid eingesetzt.

Als Träger wurden Kaninchen-IgG, Rinderserumalbumin, β-Galactosidase, Aminodextran und Rinder-Fab-Antikörperfragmente verwendet. Die Beladung des Trägers mit den Peptidsequenzen betrug 1:2-1:20 auf molarer Basis. Die Beladung des Trägers mit Markierungsgruppen betrug 1:1 bis 1:20 auf molarer Basis.

<u>Beispiel 3</u>

Herstellung von trägergebundenen multimeren Antigenen (Polyhaptenen) mit Polypeptidepitopen am Beispiel von Poly-p24-RSA-BPRu

1. Prinzip

Rinderserumalbumin (RSA) wurde mit Ruthenium- (bis-pyridyl) $_3{}^{2+}$-N-hydroxysuccinimidester (BPRU) und Maleinimidohexanoyl-N-hydroxy-succinimidester (MHS) in der angegebenen Reihenfolge umgesetzt und zur Abtrennung der freien, nicht gebundenen Derivatisierungsreagenzien jeweils dialysiert.

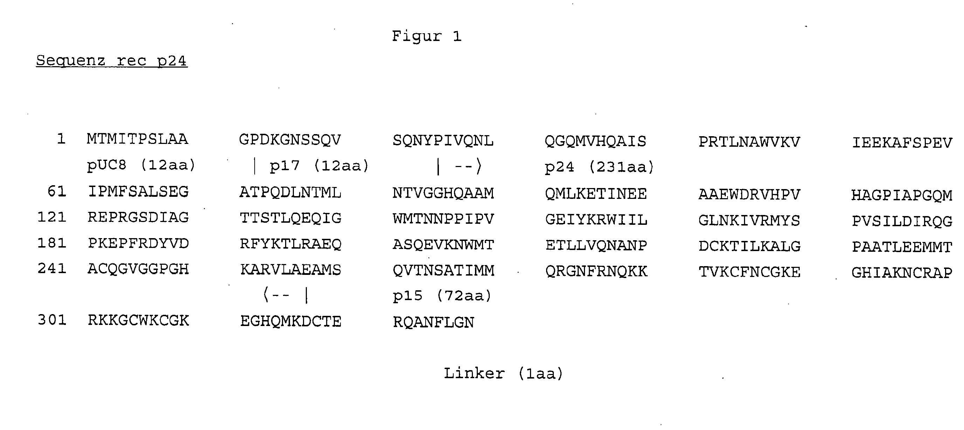

Rekombinantes p24-Antigen aus E. coli (Ghrayeb und Chang, DNA5 (1986), 93-99) mit der in Figur 1 gezeigten Aminosäuresequenz wurde mit N-Succinimidyl-S-acetylthiopropionat (SATP) zur Einführung von Thiolresten über Aminogruppen umgesetzt und zur Abtrennung von freiem, nicht gebundenen SATP dialysiert.

Nach Freisetzung der SH-Gruppen im aktivierten p24-Antigen wurden an die Maleinimido-Funktionen von RSA-BPRU gekoppelt. Überschüssige funktionelle Kopplungsgruppen wurden mit Cystein und N-Methylmaleinimid abgefangen und damit die Reaktion gestoppt.

Aus dem Reaktionsgemisch wurde dann das Produkt durch Chromatographie an Sephacryl S 200 isoliert.

2.1 Herstellung von RSA (MH)-BPRU

Zu 250 mg RSA wurde bei einer Proteinkonzentration 20 mg/ml in PBS-Puffer pH 8,0 ein 5fach molarer Überschuß von BPRU-Reagenz (0,4 ml BPRU-Stammlösung mit 47 mg/ml in DMSO) zugesetzt.

Nach Zugabe wurde 75 min bei 25°C weitergerührt. Die Reaktion wurde dann durch Zugabe von Lysin auf eine Endkonzentration von 10 mmol/l gestoppt und 30 min bei 25°C weitergerührt.

Durch Zugabe von Jodacetamid auf eine Endkonzentration von 10 mmol/l wurden vorhandene SH-Gruppen von RSA derivatisiert. Dazu wurde der Ansatz 45 min bei 25°C und pH 8,0 weitergerührt.

Freie, nicht gebundene Derivatisierungsreagenzien wurden durch Dialyse (20 Std, 4°C) gegen >500faches Volumen PBS-Puffer pH 7,5 (50 mmol/l Na-Phosphat, 150 mmol/l NaCl, pH 7,5) vollständig abgetrennt.

Der Einbau von BPRU betrug 4,7 Mol pro Mol RSA. Die Ausbeute war 220 mg RSA-BPRU (89%).

Zu 220 mg RSA-BPRU wurde dann bei einer Proteinkonzentration von 20 mg/ml in PBS-Puffer pH 7,1 ein 25fach molarer Überschuß von MHS-Reagenz zugesetzt (0,5 ml MHS-Stammlösung mit 50 mg/ml in DMSO) und 60 min bei 25°C weitergerührt.

Die Reaktion wurde durch Zugabe von Lysin auf eine Endkonzentration von 10 mmol/l gestoppt und 30 min bei 25°C weitergerührt.

Freies, nicht gebundenes MHS-Reagenz wurde durch Dialyse (20 Std, 4°C) gegen > 500-faches Volumen PBS Puffer pH 7,5 vollständig abgetrennt. Ausbeute: 210 mg RSA(MH)-BPRU (84%).

2.2 Herstellung von p24-Antigen (SATP)

Zu 100 mg p24-Antigen wurde bei einer Proteinkonzentration von 10 mg/ml in 0,1 M Na-Phosphat, 0,1% (w/v) SDS,pH 7,1, ein 3fach molarer Überschuß von SATP-Reagenz (0,06 ml SATP-Stammlösung mit 35 mg/ml in DMSO) zugesetzt und 60 min bei 25°C weitergerührt.

Die Reaktion wurde dann durch Zugabe von Lysin auf 10 mmol/l Endkonzentration gestoppt und 30 min bei 25°C weitergrührt.

Freies, nicht gebundenes SATP-Reagenz wurde anschließend durch Dialyse (20 Std., RT) gegen >500faches Volumen 0,1 mol/l Na-Phosphat, 0,1% (w/v) SDS, pH 6,5 vollständig abgetrennt. Ausbeute: 95 mg p24-Antigen (SATP) (95%).

2.3 Herstellung von Poly-p24-Antigen-RSA-BPRU

Zu 95 mg p24-Antigen (SATP) wurde bei einer Proteinkonzentration von 10 mg/ml in 0,1 mol/l Na-Phosphat, 0,1% (w/v) SDS, pH 7,5 Hydroxylamin (1 mol/l; Merck) auf 30 mmol/l Endkonzentration zugestzt und der Ansatz 60 min bei 25°C weitergerührt.

18 mg RSA(MH)-BPRU wurden zugesetzt und der Ansatz bei einer Proteinkonzentration von 9 mg/ml 60 min weitergerührt (pH 7,1; 25°C). Zum Stop wurde Cystein auf eine Endkonzentration von 2 mmol/l zugesetzt und 30 min bei pH 7,1 weitergerührt. Anschließend wurde N-Methylmaleimid (Sigma) auf eine Endkonzentration von 5 mmol/l zugesetzt und weitere 30 min bei pH 7,1 und 25°C gerührt.

Der so abgestoppte Ansatz wurde 18 Std bei Raumtemperatur (RT) gegen >500faches Volumen 0,1 mol/l Na-Phosphat, 0,1% (w/v).SDS, pH 6,5 dialysiert und über eine Sephacryl S 200-Säule (Pharmacia) gereinigt. Die wichtigsten Rahmenbedingungen für den Säulenlauf: Säulenvolumen 340 ml, Auftragsvolumen 12 ml, Flußrate 13,0 cm/Std, Laufpuffer 0,1 mol/l Na-Phosphat, 0,1% (w/v) SDS, pH 6,5, Betriebstemperatur RT.

Der Säulenlauf wurde über ein Durchflußphotometer bei einer Wellenlänge von 280 nm verfolgt und in Fraktionen gesammelt (Fraktionsgröße etwa 0,5% des Säulenvolumens).

Die Fraktionen des hochmolekularen Elutionsprofils wurden nach UV-Aufzeichnung zu einem Pool gesammelt, das Produkt in einer Amicon-Rührzelle mit YM30 Membran (Amicon) auf eine Proteinkonzentration von 10 mg/ml aufkonzentriert und bei -80°C eingefroren.

Einbau: 5 Mol p24-Antigen pro Mol p24-Antigen-RSA-BPRU. Ausbeute:19 mg.

<u>Beispiel 4</u>

Verbesserung der Sensitvität des Brückentestformats bei Verwendung von multimeren Antigenen

a) Trägergebundene multimere Antigene (Polyhaptene)

Es wurden verschiedene Varianten von biotinylierten Polyhaptenen in einem Doppelantigen-Immunoassay in Kombination mit einem monomeren digoxgenylierten Hapten eingesetzt, und zwar mit gleicher molarer Menge von biotinyliertem bzw. digoxigenyliertem Hapten. Als Epitop wurde die Aminosäuresequenz NNTRKSISIGPGRAFYT aus dem gp120-Bereich von HIV verwendet. Die Herstellung der Haptene erfolgte wie in den Beispielen 1 und 2 beschrieben. Es wurde die relative Reaktivität von nativen Anti-HIV Seren mit den verschiedenen biotinylierten Polyhaptenen auf die Reaktivität der Seren mit dem entsprechenden biotinylierten monomeren Hapten (=100% Reaktivität) normiert.

Die Ergebnisse dieses Versuchs sind in Tabelle 4 dargestellt.

| Trägermolekül | effektive Beladung pro Trägermolekül | | Reaktivität: Vgl. mit monomerem Antigen (= 100 %) |
|---|---|---|---|
| | Biotin | Peptid | |
| RSA (MW:69000) (ca. 627 Aa) | 1<br>1 | 4.2<br>5.1 | ca. 173,0 %<br>ca. 185,0 % |
| β-Gal (MW:465000) (ca. 4227 Aa) | 1<br>1<br>1 | 2.2<br>3.6<br>9.4 | ca. 123.5 %<br>ca. 151,8 %<br>ca. 125,0% |
| Rinder-Fab (MW:75000) (ca. 682 Aa) | 1 | 5.9 | ca. 146,0 % |

b) Multimere verzweigte Antigene

Es wurde ein Vergleich von biotinylierten und ruthenylierten Antigenen mit monomeren bzw. multimeren verzweigten Epitopen in einem Doppelantigen-Immunoassy im Brückentestformat durchgeführt.

Bei einem Epitop aus der NS4-Region von HCV (Sequenz SRGNHVSPTHYVPESDAA) wurde die Kombination eines monomeren biotinylierten Antigens und eines monomeren ruthenylierten Antigens mit der Kombination eines multimeren verzweigten biotinylierten Antigens (siehe Tabelle 3d, Zeile 2) und eines monomeren ruthenylierten Antigens in einem Brückentest verglichen. Es wurde die Signaldifferenzierung, d.h. das Verhältnis im Meßsignal zwischen positiven und negativen Proben bestimmt. Eine höhere Signaldifferenzierung bedeutet eine bessere Sensitivität. Bei Verwendung eines multimeren biotinylierten Antigens wurde eine Signaldifferenzierung von 386 gegenüber einer Signaldifferenzierung von nur 208 bei der Kombination beider monomerer Antigene erhalten.

Entsprechend wurde ein Doppelantigen-Brückentest mit einer Antigensequenz aus der gp120-Region von HIV durchgeführt. Das verwendete Epitop hatte die Aminosäuresequenz NTTRSISIGPGRAFY. Es wurde die Kombination eines monomeren biotinylierten und eines monomeren ruthenylierten Antigens mit der Kombination eines multimeren verzweigten biotinylierten Antigens (siehe Tabelle 2d, Zeile 2) und eines multimeren ruthenylierten Antigens (siehe Tabelle 2d, Zeile 4) verglichen. Bei einem Test mit der Kombination der beiden multimeren Antigene wurde eine Signaldifferenzierung zwischen positiver und negativer Probe von 12 gefunden. Die Kombination beider monomerer Antigene zeigte hingegen nur eine Signaldifferenzierung von 10.

Beispiel 5

Verbesserung der Sensitivität des Brückentestformats bei Verwendung von multimeren trägergebundenen Antigenen

Es wurde die Kombination eines monomeren biotinylierten und eines monomeren ruthenylierten Antigens mit der Kombination aus einem monomeren biotinylierten Antigen und einem trägergebundenen multimeren ruthenylierten Antigen (Trägermolekül: Rinderserumalbumin; Epitop: HIV-p24-Antigen; Herstellung gemäß Beispiel 3) und mit der Kombination eines trägergebundenen multimeren biotinylierten Antigens und eines monomeren ruthenylierten Antigens in einem Brückentest untersucht. Bei zwei unterschiedlichen positiven Proben (HIV-Seren) wurde bei der Kombination der beiden monomeren Antigene jeweils eine Signaldifferenzierung positiv/negativ von 2 gefunden, während die Kombination von monomerem biotinyliertem Antigen und multimerem ruthenyliertem Antigen eine Differenzierung von 19 bzw. 7 und die Kombination von multimerem biotinyliertem Antigen und monomerem ruthenyliertem Antigen eine Differenzierung von 4 bzw 3 ergab.

Auch bei Verwendung einer Kombination eines monomeren biotinylierten Antigens und eines anderen multimeren ruthenylierten Antigens (Trägermolekül: Kaninchen-Immunglobulin) wurde bei drei verschiedenen positiven Proben eine wesentlich größere Signaldifferenzierung positiv/negativ von 3,22 bzw. 10 gegenüber 2,9 bzw 8 bei einer Kombination der monomeren Antigene gefunden.

Auch bei Verwendung eines anderen Epitops (Protein aus dem HIV-gp41-Bereich) konnte die Überlegenheit der multimeren Antigene gegenüber den monomeren Antigenen gezeigt werden. Während bei einer Kombination aus monomeren biotinylierten und digoxigenylierten Antigenen im Brückentest praktisch keine Differenzierung zwischen negativ und positiv gefunden wurde, zeigte die Kombination aus multimeren Polyhaptenen eine sehr gute Differenzierung.

Beispiel 6

Verbesserung der Sensitivität des Brückentestformats bei Verwendung von multimeren Antigenen

Insbesondere die Kombination wandseitiges monomeres Antigen und markiertes multimeres Antigen ist zum Erreichen einer optimalen Sensitivität in einem breiten Konzentrationsbereich an spezifischem Immunglobulin bevorzugt. Die bevorzugten Einsatzmengen sind 1 Äquivalent wandseitiges Epitop zu 0,2 - 10 und insbesondere zu 0,2 - 8 Äquivalente markierte Epitope.

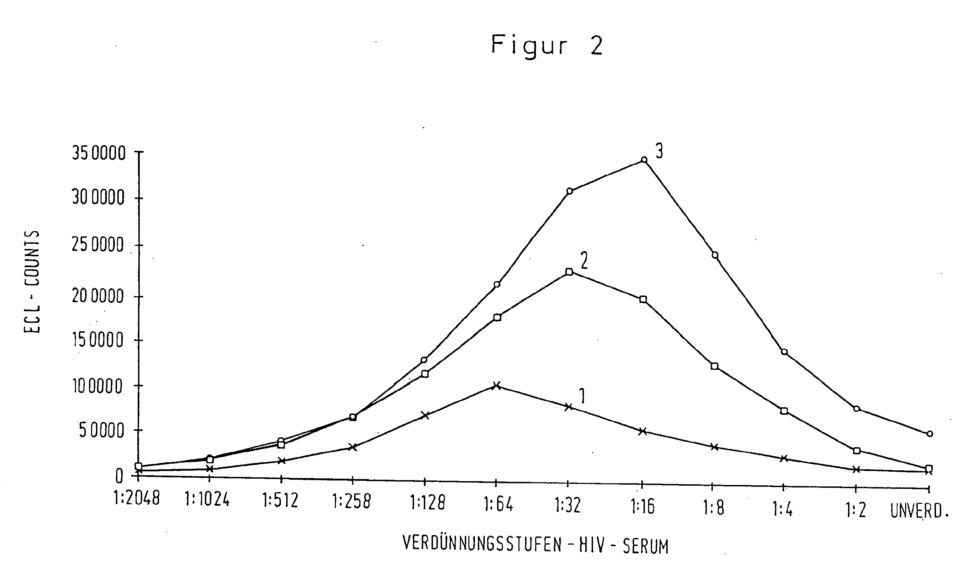

Figur 2 zeigt einen Vergleich der Kombination aus einem monomeren biotinylierten Antigen und einem monomeren ruthenylierten Antigen in einem Epitopverhältnis 1:1 (Kurve 1) und der Kombination aus einem monomeren biotinylierten Antigen und einem multimeren ruthenylierten trägergebundenen Antigen in einem Epitopverhältnis von 1:2 (Kurve 2) bzw. 1:4 (Kurve 3).

Als Epitop wurde die in Beispiel 4a angegebene Sequenz aus dem gp120-Bereich von HIV verwendet. Das Trägermolekül des multimeren Antigens war RSA. Die Beladung des Trägers war mit den Epitopgruppen 5:1 und mit den BPRu-Gruppen 3:1 jeweils auf molarer Basis.

Aus Fig. 2 ist ersichtlich; daß die Verwendung von multimeren Antigenen zu einer Verringerung des Hook-Effekts und zu einer allgemeinen Sensitivitätssteigerung führt.

Beispiel 7

Verbesserung der Sensitivität des Brückentestformats bei Verwendung von multimeren Antigenen durch Erhöhung der Anzahl von Markierungsgruppen

Eine weitere Verbesserung der Testsensitivität wird dadurch erreicht, daß eine Erhöhung der Anzahl von Markierungs- bzw. Festphasenbindegruppen in größerem Umfang möglich ist, ohne die Epitopbereiche zu maskieren oder durch Erhöhung der Hydrophobizität die unspezifischen Hintergrundwerte zu erhöhen.

Es wurden digoxigenylierte multimere Antigene verglichen, die Epitope aus dem gp120-Bereich von HIV (vgl. Beispiel 4b) gekoppelt auf einem Rinder-Fab-Antikörperfragment-Träger enthielten. Die Beladung des Trägers mit dem Peptidepitop war jeweils im Bereich von 1:6 bis 1:7 auf molarer Basis. Die Beladung des Trägers mit Digoxigeningruppen war 1:2 bzw. 1:4.

Die Ergebnisse dieses Versuchs sind in Tabelle 5 gezeigt. Es ist zu erkennen, daß durch Erhöhung der Anzahl von Markierungsgruppen eine nicht-lineare Verbesserung der Sensitvität und eine erhebliche Verringerung des Hook-Effekts erreicht wurde.

Tabelle 5

| Probe | Stöchiometrie Träger: Dig. 1:4 | Stöchiometrie Träger: Dig. 1:2 |
|---|---|---|
| **Dynamik des Meßbereichs** Verdünnungsstufen | mE | mE |
| 1/16384 | 36 | 148 |
| 1/8192 | 48 | 159 |
| 1/4096 | 49 | 151 |
| 1/2048 | 82 | 158 |
| 1/1024 | 132 | 159 |
| 1/512 | 302 | 157 |
| 1/256 | 675 | 164 |
| 1/128 | 1503 | 190 |
| 1/64 | 3493 | 259 |
| 1/32 | 7436 | 480 |
| 1/16 | 9306 | 1066 |
| 1/8 | 9449 | 3036 |
| 1/4 | 9449 | 3378 |
| 1/2 | 9449 | 2694 |
| unverdünnt | 9474 | 2266 |

Beispiel 8

Verbesserung der Stabilität durch Verwendung multimerer Antigene

Es wurde die Stabilität von monomeren und multimeren Antigenen getestet. Hierzu wurde die Signalwiederfindung nach dreitägiger Inkubation bei 35°C bezogen auf die ursprüngliche Signalintensität bestimmt.

Für ein monomeres ruthenyliertes Antigen aus dem gp120-Bereich von HIV (Sequenz siehe Beispiel 4) wurde in Kombination mit einem frischen biotinylierten monomeren Antigen eine Signalwiederfindung von 3,0 bzw 4,0 % bei zwei Proben ermittelt. Bei Verwendung eines trägergebundenen multimeren ruthenylierten Antigens (Träger: Kaninchen-IgG, 4 Markierungsgruppen und 3 Epitope pro Trägermolekül) wurden unter gleichen Testbedingungen Signalwiederfindungen von 73,1 bzw 73,6 % ermittelt.

Auf entsprechende Weise wurde ein biotinyliertes monomeres Antigen mit der gleichen Epitopsequenz mit einem trägergebundenen biotinylierten multimeren Antigen (Träger: Kaninchen-IgG, 18 Biotingruppen und 3 Epitope pro Träger) in Kombination mit einem monomeren ruthenylierten Antigen untersucht. Für das monomere biotinylierte Antigen wurden Signalwiederfindungen von 25,0 bzw. 37,0 % und für das multimere Antigen Signalwiederfindungen von 120,3 bzw. 79,9 % ermittelt.

Beispiel 9

Verbesserung der Sensitivität bezüglich der Reaktivität mit Antigenen niedriger Affinität

Multimere Antigene werden bevorzugt für die Erkennung von spezifischem Immunglobulin mit niedriger Affinität, z.B. bei einer vor kurzen erfolgten Serokonversion und bei neuen viralen Subtypen eingesetzt.

a) Ruthenylierte multimere Antigene

Es wurde die Signaldifferenzierung positiv/negativ unter Verwendung von Antigenen mit einer Epitopsequenz aus dem NS4/3-Bereich von HCV untersucht. Die Kombination eines monomeren ruthenylierten und eines monomeren biotinylierten Antigens ergab bei zwei verschiedenen positiven Serokonversionsproben eine Signaldifferenzierung positv/negativ von 3 bzw 1, d.h. eine positive Probe wurde nicht als solche erkannt. Bei Verwendung von multimeren IgG-trägergebundenen biotinylierten und ruthenylierten Antigenen wurde eine Signaldifferenzierung von jeweils 21 ermittelt. Erst durch Verwendung multimerer Antigene können die positiven Proben richtig klassifiziert werden.

b) Biotinylierte und digoxigenylierte multimere Antigene

Es wurden das jeweils gleiche Peptidepitop aus dem gp41-Bereich von HIV (gp41/3) als trägergebundenes multimeres Antigen und als monomeres Antigen gegenübergestellt. Es wurden jeweils 50 ng/ml biotinyliertes und digoxigenyliertes monomeres Peptid eingesetzt. Bei den multimeren Antigenen wurden 50 ng/ml "Peptidäquivalent" eingesetzt, wobei die Peptidmenge über den Beladungsgrad des Polyhaptens berechnet wurde. Der Test wurde an Analysenautomaten ES700 durchgeführt.

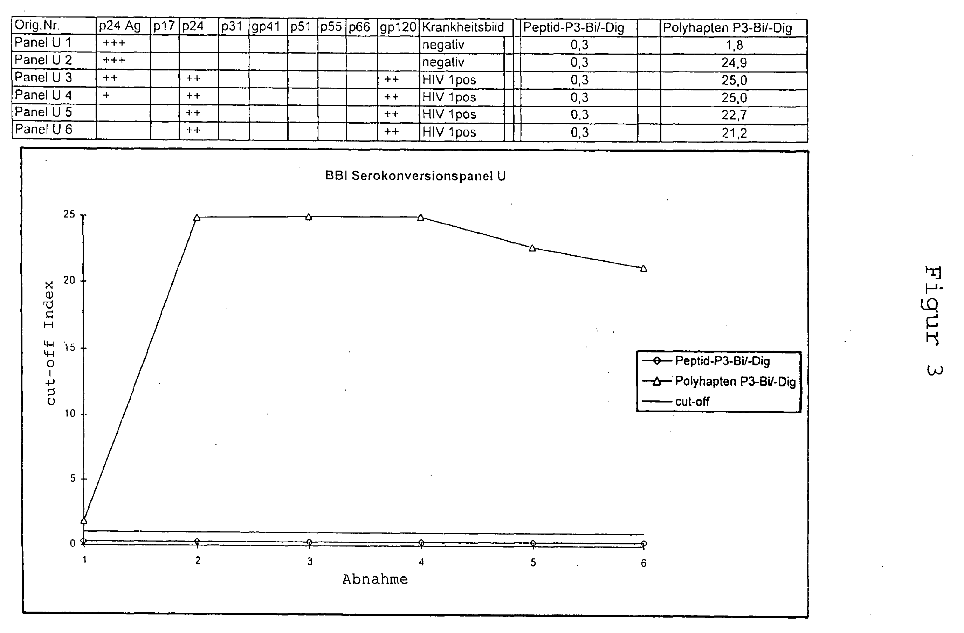

Die Test wurden unter Verwendung von unterschiedlichen Serokonversionspanels als Proben durchgeführt. Fig. 3 zeigt, daß die Panels bei Tests unter Verwendung des digoxigenylierten Polyhaptens als korrekt positiv klassifiziert wurden, während bei Verwendung des monomeren Antigens ein falsch negatives Resultat erhalten wurde.
Der "cut-off"-Index ist die Grenze zwischen negativer und positiver Bewertung eines Experiments. Er ist als 2x den Wert der negativen Kontrolle definiert.

**Patentansprüche**

1. Verfahren zur immunologischen Bestimmung eines spezifischen Antikörpers in einer Probeflüssigkeit, wobei man die Probeflüssigkeit in Gegenwart einer Festphase mit zwei gegen den zu bestimmenden Antikörper gerichteten Antigenen inkubiert, wobei das erste Antigen mindestens eine Markierungsgruppe trägt und das zweite Antigen (a) an die Festphase gebunden ist oder (b) in einer an die Festphase bindefähigen Form vorliegt, und den zu bestimmenden Antikörper durch Bestimmung der Markierungsgruppe in der Festphase oder/und in der flüssigen Phase nachweist,
**dadurch gekennzeichnet,**
**daß** mindestens eines der beiden Antigene mehrere Epitopbereiche umfaßt, die mit dem zu bestimmenden Antikörper reagieren.

2. Verfahren nach Anspruch 1,
**dadurch gekennzeichnet,**
**daß** mindestens eines der beiden Antigene mehrere gleiche Epitopbereiche umfaßt.

3. Verfahren nach Anspruch 1 oder 2,
**dadurch gekennzeichnet,**
**daß** das erste markierte Antigen mehrere Epitopbereiche umfaßt.

4. Verfahren nach Anspruch 1 oder 2,
**dadurch gekennzeichnet,**
**daß** das zweite festphasenseitige Antigen mehrere Epitopbereiche umfaßt.

5. Verfahren nach Anspruch 1 oder 2,
**dadurch gekennzeichnet,**
**daß** das erste markierte Antigen und das zweite festphasenseitige Antigen mehrere Epitopbereiche umfassen.

6. Verfahren nach einem der Ansprüche 1 bis 5,
**dadurch gekennzeichnet,**
**daß** das erste markierte Antigen mindestens eine Metallchelat-Markierungsgruppe trägt.

7. Verfahren nach einem der Ansprüche 1 bis 5,
**dadurch gekennzeichnet,**
**daß** das erste markierte Antigen mindestens eine Hapten-Markierungsgruppe trägt und daß man die Probeflüssigkeit weiterhin mit einem Rezeptor für das Hapten inkubiert, der eine signalerzeugende Gruppe trägt.

8. Verfahren nach Anspruch 7,
**dadurch gekennzeichnet,**
**daß** man das Hapten aus der Gruppe bestehend aus Sterinen, Gallensäuren, Sexualhormonen, Corticoiden, Cardenoliden, Cardenolid-Glycosiden, Bufadienolen, Steroid-Sapogeninen und Steroidalkaloiden auswählt und daß man als Rezeptor einen Antikörper verwendet, der gegen das Hapten gerichtet ist.

9. Verfahren nach einem der Ansprüche 1 bis 8,
**dadurch gekennzeichnet,**
**daß** das zweite festphasenseitige Antigen biotinyliert ist und die Festphase mit Streptavidin oder Avidin beschichtet ist.

10. Verfahren nach einem der Ansprüche 1 bis 9,
**dadurch gekennzeichnet,**
**daß** man ein Antigen verwendet, das einen nicht mit dem zu bestimmenden Antikörper reagierenden Träger umfaßt, an den mehrere Epitopbereiche kovalent gekoppelt sind.

11. Verfahren nach Anspruch 10,
**dadurch gekennzeichnet,**
**daß** man als Träger ein Peptid, ein Polypeptid oder einen synthetischen Träger verwendet.

12. Verfahren nach Anspruch 11,

**dadurch gekennzeichnet,**
**daß man als Träger ein Polypeptid, ausgewählt aus der** Gruppe bestehend aus Albuminen, Immunglobulinen, Immunglobulin-Fragmenten und β-Galactosidase, verwendet.

**13.** Verfahren nach einem der Ansprüche 10 bis 12,
**dadurch gekennzeichnet,**
**daß** man ein Antigen der allgemeinen Formel verwendet:

$$(P-)_nT(-L)_m \quad (Ia)$$

oder

$$T(-P-L_m)_n \quad (Ib)$$

wobei T einen Träger bedeutet, P Peptid- oder Polypeptidsequenzen bedeutet, die gleiche oder verschiedene, immunologisch reaktive Epitopbereiche enthalten und kovalent an den Träger gekoppelt sind, und L Markierungsgruppen oder zur Bindung an eine Festphase fähige Gruppen bedeutet, die kovalent an den Träger bzw. an die Peptid- oder Polypeptidsequenzen gekoppelt sind, n eine Zahl von größer 1 bis 40 ist und m eine Zahl von 1 bis 10 ist.

**14.** Verfahren nach Anspruch 13,
**dadurch gekennzeichnet,**
**daß** P synthetische Peptidsequenzen mit einer Länge von 6 bis 50 Aminosäuren bedeutet, die neben den Epitopbereichen noch gegebenenfalls immunologisch inaktive Spacerbereiche umfassen.

**15.** Verfahren nach Anspruche 13,
**dadurch gekennzeichnet,**
**daß** P rekombinante Polypeptidsequenzen mit einer Länge bis zu 1000 Aminosäuren bedeutet.

**16.** Verfahren nach einem der Ansprüche 1 bis 9,
**dadurch gekennzeichnet,**
**daß** man ein Antigen verwendet, das mehrere direkt oder über Spacerbereiche miteinander kovalent gekoppelte Epitopbereiche umfaßt.

**17.** Verfahren nach Anspruch 16,
**dadurch gekennzeichnet,**
**daß** man ein Antigen mit der allgemeinen Formel (II) verwendet:

$$P^1\{P^2[P^3(P^4)_t]_s\}_r \qquad (II)$$

wobei $P^1$, $P^2$, $P^3$ und $P^4$ Peptidsequenzen mit einer Länge von bis zu 50 Aminosäuren bedeuten, wobei mindestens 2 Peptidsequenzen gleiche oder verschiedene, immunologisch reaktive Epitopbereiche enthalten, r 1 oder 2 ist, s eine ganze Zahl von 0 bis 4 ist und t eine ganze Zahl von 0 bis 8 ist, wobei das Antigen mindestens eine Verzweigungsstelle und mindestens eine Markierungsgruppe oder eine zur Bindung an eine Festphase fähige Gruppe enthält.

**18.** Verfahren nach Anspruch 17,
**dadurch gekennzeichnet,**
**daß** die Verzweigungsstellen durch trifunktionelle Aminosäuren gebildet sind.

**19.** Verfahren nach Anspruch 18,
**dadurch gekennzeichnet,**
**daß** die Verzweigungsstellen durch Lysin oder/und Ornithin gebildet sind.

**20.** Verfahren nach einem der Ansprüche 1 bis 9,
**dadurch gekennzeichnet,**
**daß** man als Antigen, das mehrere Epitopbereiche umfaßt, ein rekombinantes Fusionspolypeptid verwendet, dessen Aminosäuresequenz mehrere immunologisch reaktive Epitopbereiche enthält, die über immunologisch inaktive Spacerbereiche verknüpft sein können.

**21.** Reagenz zur immunologischen Bestimmung eines spezifischen Antikörpers in einer Probeflüssigkeit,
umfassend
eine reaktive Festphase,
zwei gegen den zu bestimmenden Antikörper gerichtete Antigene, wobei das erste Antigen eine Markierungsgruppe trägt und das zweite Antigen (a) an die Festphase gebunden ist oder (b) in einer an die Festphase bindefähigen Form vorliegt,
**dadurch gekennzeichnet,**
**daß** mindestens eines der beiden Antigene mehrere Epitopbereiche umfaßt, die mit dem zu bestimmenden Antikörper reagieren.

**22.** Reagenz nach Anspruch 21, umfassend ein erstes markiertes Antigen mit mehreren Epitopbereichen, das mindestens eine Hapten-Markierungsgruppe trägt, und einen Rezeptor für das Hapten, der eine signalerzeugende Gruppe enthält.

**23.** Reagenz nach Anspruch 21 oder 22, umfassend ein zweites festphasenseitiges Antigen mit mehreren Epitopbereichen, das mindestens eine Biotingruppe trägt, und eine mit Streptavidin oder Avidin beschichtete reaktive Festphase.

**24.** Verwendung von multimeren Antigenen, die mehrere immunologisch reaktive Epitopbereiche umfassen, in einem immunologischen Testverfahren zur Bestimmung eines spezifischen Antikörpers.

**25.** Antigen der allgemeinen Formel

$$(P-)_nT(-L)_m \quad (Ia)$$

oder

$$T(-P-L_m)_n \quad (Ib)$$

wobei T einen Träger bedeutet, P Peptid- oder Polypeptidsequenzen bedeutet, die gleiche oder verschiedene, immunologisch reaktive Epitopbereiche enthalten und kovalent an den Träger gekoppelt sind, und L Markierungsgruppen oder zur Bindung an eine Festphase fähige Gruppen bedeutet, die kovalent an den Träger bzw. an die Peptid- oder Polypeptidsequenzen gekoppelt sind, n eine Zahl von größer 1 bis 40 ist und m eine Zahl von 1 bis 10 ist.

**26.** Antigen der allgemeinen Formel

$$P^1\{P^2[P^3(P^4)_t]_s\}_r \quad (II)$$

wobei $P^1$, $P^2$, $P^3$ und $P^4$ Peptidsequenzen mit einer Länge von bis zu 50 Aminosäuren bedeuten, wobei mindestens 2 Peptidsequenzen gleiche oder verschiedene immunologisch reaktive Epitopbereiche enthalten, r 1 oder 2 ist, s eine ganze Zahl von 0 bis 4 ist und t eine ganze Zahl von 0 bis 8 ist, wobei Antigen mindestens eine Verzweigungsstelle und mindestens eine Markierungsgruppe oder eine zur Bindung an eine Festphase fähige Gruppe enthält.

Figur 1

<u>Sequenz rec p24</u>

```
  1  MTMITPSLAA   GPDKGNSSQV   SQNYPIVQNL   QGQMVHQAIS   PRTLNAWVKV   IEEKAFSPEV
     pUC8 (12aa)   | p17 (12aa)     | --⟩    p24 (231aa)
 61  IPMFSALSEG   ATPQDLNTML   NTVGGHQAAM   QMLKETINEE   AAEWDRVHPV   HAGPIAPGQM
121  REPRGSDIAG   TTSTLQEQIG   WMTNNPPIPV   GEIYKRWIIL   GLNKIVRMYS   PVSILDIRQG
181  PKEPFRDYVD   RFYKTLRAEQ   ASQEVKNWMT   ETLLVQNANP   DCKTILKALG   PAATLEEMMT
241  ACQGVGGPGH   KARVLAEAMS   QVTNSATIMM   QRGNFRNQKK   TVKCFNCGKE   GHIAKNCRAP
                 ⟨-- |       p15 (72aa)
301  RKKGCWKCGK   EGHQMKDCTE   RQANFLGN

                                   Linker (1aa)
```

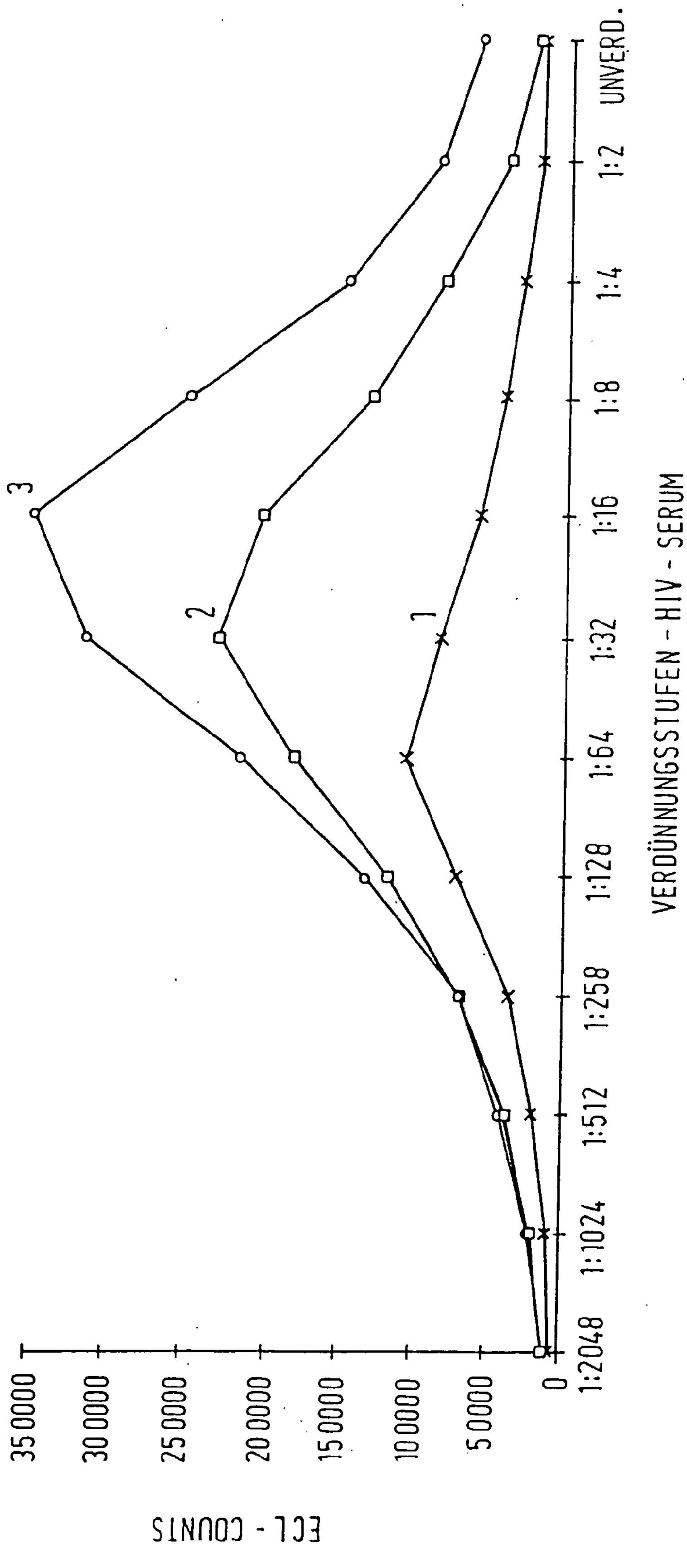
ECL - COUNTS
35 0000
30 0000
25 0000
20 0000
15 0000
10 0000
5 0000
0
1:2048
1:1024
1:512
1:258
1:128
1:64
1:32
1:16
1:8
1:4
1:2
UNVERD.
VERDÜNNUNGSSTUFEN - HIV - SERUM
1
2
3

Figur 2

# Figur 3

| Orig.Nr. | p24 Ag | p17 | p24 | p31 | gp41 | p51 | p55 | p66 | gp120 | Krankheitsbild | Peptid-P3-Bi/-Dig | Polyhapten P3-Bi/-Dig |
|---|---|---|---|---|---|---|---|---|---|---|---|---|
| Panel U 1 | +++ | | | | | | | | | negativ | 0,3 | 1,8 |
| Panel U 2 | +++ | | | | | | | | | negativ | 0,3 | 24,9 |
| Panel U 3 | ++ | | ++ | | | | | | ++ | HIV 1pos | 0,3 | 25,0 |
| Panel U 4 | + | | ++ | | | | | | ++ | HIV 1pos | 0,3 | 25,0 |
| Panel U 5 | | | ++ | | | | | | ++ | HIV 1pos | 0,3 | 22,7 |
| Panel U 6 | | | ++ | | | | | | ++ | HIV 1pos | 0,3 | 21,2 |

SEQUENZPROTOKOLL

(1) ALLGEMEINE INFORMATION:

(i) ANMELDER:

(A) NAME: Boehringer Mannheim GmbH

(B) STRASSE: Sandhofer Str. 116

(C) ORT: Mannheim

(E) LAND: Deutschland

(F) POSTLEITZAHL: 68305

(ii) ANMELDETITEL: Bestimmung von spezifischem Immunglobulin unter Verwendung multipler Antigene

(iii) ANZAHL DER SEQUENZEN: 17

(iv) COMPUTER-LESBARE FORM:

(A) DATENTRÄGER: Floppy disk

(B) COMPUTER: IBM PC compatible

(C) BETRIEBSSYSTEM: PC-DOS/MS-DOS

(D) SOFTWARE: PatentIn Release #1.0, Version #1.25 (EPA)

(2) INFORMATION ZU SEQ ID NO: 1:

(i) SEQUENZ CHARAKTERISTIKA:

(A) LÄNGE: 17 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(viii) POSITION IM GENOM:

(A) CHROMOSOM/SEGMENT: gp120

(xi) SEQUENZBESCHREIBUNG: SEQ ID NO: 1:

```
Asn Asn Thr Arg Lys Ser Ile Ser Ile Gly Pro Gly Arg Ala Phe Tyr
1               5                   10                  15

Thr
```

(2) INFORMATION ZU SEQ ID NO: 2:

(i) SEQUENZ CHARAKTERISTIKA:

(A) LÄNGE: 16 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(viii) POSITION IM GENOM:

(A) CHROMOSOM/SEGMENT: gp120

(xi) SEQUENZBESCHREIBUNG: SEQ ID NO: 2:

Asn Thr Thr Arg Ser Ile Ser Ile Gly Pro Gly Arg Ala Phe Tyr Thr
1 5 10 15

(2) INFORMATION ZU SEQ ID NO: 3:

(i) SEQUENZ CHARAKTERISTIKA:

(A) LÄNGE: 19 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(B) STAMM: Subtype O

(viii) POSITION IM GENOM:

(A) CHROMOSOM/SEGMENT: gp120

(xi) SEQUENZBESCHREIBUNG: SEQ ID NO: 3:

Ile Asp Ile Gln Glu Glu Arg Arg Met Arg Ile Gly Pro Gly Met Ala
1 5 10 15

Trp Tyr Ser

(2) INFORMATION ZU SEQ ID NO: 4:

(i) SEQUENZ CHARAKTERISTIKA:

(A) LÄNGE: 24 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(viii) POSITION IM GENOM:

(A) CHROMOSOM/SEGMENT: gp41

(xi) SEQUENZBESCHREIBUNG: SEQ ID NO: 4:

```
Gln Ala Arg Ile Leu Ala Val Glu Arg Tyr Leu Lys Asp Gln Gln Leu
1               5                   10                  15

Leu Gly Ile Trp Gly Ala Ser Gly
            20
```

(2) INFORMATION ZU SEQ ID NO: 5:

(i) SEQUENZ CHARAKTERISTIKA:

(A) LÄNGE: 23 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(viii) POSITION IM GENOM:

(A) CHROMOSOM/SEGMENT: gp41

(xi) SEQUENZBESCHREIBUNG: SEQ ID NO: 5:

```
Leu Gly Ile Trp Gly Cys Ser Gly Lys Leu Ile Cys Thr Thr Ala Val
1               5                   10                  15

Pro Trp Asn Ala Ser Trp Ser
            20
```

(2) INFORMATION ZU SEQ ID NO: 6:
(i) SEQUENZ CHARAKTERISTIKA:
(A) LÄNGE: 15 Aminosäuren
(B) ART: Aminosäure
(D) TOPOLOGIE: linear
(ii) ART DES MOLEKÜLS: Peptid
(iii) HYPOTHETISCH: NEIN
(vi) URSPRÜNGLICHE HERKUNFT:
(A) ORGANISMUS: Human immunodeficiency virus type 1
(viii) POSITION IM GENOM:
(A) CHROMOSOM/SEGMENT: gp41

(xi) SEQUENZBESCHREIBUNG: SEQ ID NO: 6:

Lys Asp Gln Gln Leu Leu Gly Ile Trp Gly Ser Ser Gly Lys Leu
1 5 10 15

(2) INFORMATION ZU SEQ ID NO: 7:
(i) SEQUENZ CHARAKTERISTIKA:
(A) LÄNGE: 15 Aminosäuren
(B) ART: Aminosäure
(D) TOPOLOGIE: linear
(ii) ART DES MOLEKÜLS: Peptid
(iii) HYPOTHETISCH: NEIN
(vi) URSPRÜNGLICHE HERKUNFT:
(A) ORGANISMUS: Human immunodeficiency virus type 1
(B) STAMM: Subtype O
(viii) POSITION IM GENOM:
(A) CHROMOSOM/SEGMENT: gp41

(xi) SEQUENZBESCHREIBUNG: SEQ ID NO: 7:

Ala Leu Glu Thr Leu Leu Gln Asn Gln Gln Leu Leu Ser Leu Trp
1 5 10 15

(2) INFORMATION ZU SEQ ID NO: 8:

(i) SEQUENZ CHARAKTERISTIKA:

(A) LÄNGE: 15 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(B) STAMM: Subtype O

(viii) POSITION IM GENOM:

(A) CHROMOSOM/SEGMENT: gp41

(xi) SEQUENZBESCHREIBUNG: SEQ ID NO: 8:

Leu Ser Leu Trp Gly Cys Lys Gly Lys Leu Val Cys Tyr Thr Ser
1 5 10 15

(2) INFORMATION ZU SEQ ID NO: 9:

(i) SEQUENZ CHARAKTERISTIKA:

(A) LÄNGE: 19 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(B) STAMM: Subtype O

(viii) POSITION IM GENOM:

(A) CHROMOSOM/SEGMENT: gp41

(xi) SEQUENZBESCHREIBUNG: SEQ ID NO: 9:

Trp Gly Ile Arg Gln Leu Arg Ala Arg Leu Leu Ala Leu Glu Thr Leu
1 5 10 15

Leu Gln Asn

(2) INFORMATION ZU SEQ ID NO: 10:

(i) SEQUENZ CHARAKTERISTIKA:

(A) LÄNGE: 27 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Human immunodeficiency virus type 1

(B) STAMM: Subtype O

(viii) POSITION IM GENOM:

(A) CHROMOSOM/SEGMENT: gp32

(xi) SEQUENZBESCHREIBUNG: SEQ ID NO: 10:

```
Gln Ala Gln Leu Asn Ser Trp Gly Cys Ala Phe Arg Gln Val Cys His
1               5                   10                  15

Thr Thr Val Pro Trp Pro Asn Asp Ser Leu Thr
            20                  25
```

(2) INFORMATION ZU SEQ ID NO: 11:

(i) SEQUENZ CHARAKTERISTIKA:

(A) LÄNGE: 15 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C virus

(viii) POSITION IM GENOM:

(A) CHROMOSOM/SEGMENT: NS5

(xi) SEQUENZBESCHREIBUNG: SEQ ID NO: 11:

```
Ser Arg Arg Phe Ala Gln Ala Leu Pro Val Trp Ala Arg Pro Asp
1               5                   10                  15
```

(2) INFORMATION ZU SEQ ID NO: 12:

(i) SEQUENZ CHARAKTERISTIKA:

(A) LÄNGE: 16 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C virus

(viii) POSITION IM GENOM:

(A) CHROMOSOM/SEGMENT: Core

(xi) SEQUENZBESCHREIBUNG: SEQ ID NO: 12:

```
Pro Gln Asp Val Lys Phe Pro Gly Gly Gly Gln Ile Val Gly Gly Val
1               5                   10                  15
```

(2) INFORMATION ZU SEQ ID NO: 13:

(i) SEQUENZ CHARAKTERISTIKA:

(A) LÄNGE: 12 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C virus

(viii) POSITION IM GENOM:

(A) CHROMOSOM/SEGMENT: NS4

(xi) SEQUENZBESCHREIBUNG: SEQ ID NO: 13:

```
Glu Glu Ala Ser Gln His Leu Pro Tyr Ile Glu Gln
1               5                   10
```

(2) INFORMATION ZU SEQ ID NO: 14:

(i) SEQUENZ CHARAKTERISTIKA:

(A) LÄNGE: 9 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C Virus

(viii) POSITION IM GENOM:

(A) CHROMOSOM/SEGMENT: NS4

(xi) SEQUENZBESCHREIBUNG: SEQ ID NO: 14:

```
Gln Lys Ala Leu Gly Leu Leu Gln Thr
1               5
```

(2) INFORMATION ZU SEQ ID NO: 15:

(i) SEQUENZ CHARAKTERISTIKA:

(A) LÄNGE: 18 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C Virus

(viii) POSITION IM GENOM:

(A) CHROMOSOM/SEGMENT: NS4

(xi) SEQUENZBESCHREIBUNG: SEQ ID NO: 15:

```
Ser Arg Gly Asn His Val Ser Pro Thr His Tyr Val Pro Glu Ser Asp
1               5                   10                  15

Ala Ala
```

(2) INFORMATION ZU SEQ ID NO: 16:

(i) SEQUENZ CHARAKTERISTIKA:

(A) LÄNGE: 28 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C Virus

(viii) POSITION IM GENOM:

(A) CHROMOSOM/SEGMENT: Core

(xi) SEQUENZBESCHREIBUNG: SEQ ID NO: 16:

```
Pro Gln Arg Lys Asn Lys Arg Asn Thr Asn Arg Arg Pro Gln Asp Val
1               5                   10                  15

Lys Phe Pro Gly Gly Gly Gln Ile Val Gly Val Val
            20                  25
```

(2) INFORMATION ZU SEQ ID NO: 17:

(i) SEQUENZ CHARAKTERISTIKA:

(A) LÄNGE: 25 Aminosäuren

(B) ART: Aminosäure

(D) TOPOLOGIE: linear

(ii) ART DES MOLEKÜLS: Peptid

(iii) HYPOTHETISCH: NEIN

(vi) URSPRÜNGLICHE HERKUNFT:

(A) ORGANISMUS: Hepatitis C Virus

(viii) POSITION IM GENOM:

(A) CHROMOSOM/SEGMENT: NS3

(xi) SEQUENZBESCHREIBUNG: SEQ ID NO: 17:

```
Ala Trp Tyr Glu Leu Thr Pro Ala Glu Thr Thr Val Arg Leu Arg Ala
1               5                   10                  15

Tyr Met Asn Thr Pro Gly Leu Pro Val
            20                  25
```